# EUROPEAN PATENT SPECIFICATION

(11) **EP 1 972 251 B1**
(45) Date of publication and mention of the grant of the patent: **10.07.2013**
(21) Application number: 06843519.7
(22) Date of filing: 27.12.2006
(51) Int. Cl.: A61B 1/00, A61B 5/07, A61B 8/12

(54) **CAPSULE CONTAINER AND ADMINISTRATION METHOD USING THE SAME**
KAPSELBEHÄLTER UND VERABREICHUNGSVERFAHREN DAMIT
CONTENANT DE TYPE CAPSULE ET PROCEDE D'ADMINISTRATION L'UTILISANT

(30) Priority: 28.12.2005 JP 2005380452
(43) Date of publication of application: 24.09.2008
(73) Proprietor: OLYMPUS MEDICAL SYSTEMS CORP., Tokyo 151-0072 (JP)
(72) Inventor: TAKIZAWA, Hironobu, Shibuya-ku, Tokyo 151-0072 (JP); KAWANO, Hironao, Shibuya-ku, Tokyo 151-0072 (JP)
(74) Representative: von Hellfeld, Axel
(86) International application number: PCT/JP2006/326137
(87) International publication number: WO 2007/074882

(56) References cited:
- WO-A1-2005/117801
- WO-A2-2005/060348
- JP-A- 2003 505 154
- JP-A- 2003 523 795
- JP-A- 2005 095 433

## Description

### TECHNICAL FIELD

The present invention relates to a capsule housing apparatus for housing a swallow-type capsule medical apparatus to be introduced into a subject

### BACKGROUND ART

Recently, capsule endoscopes equipped with an imaging function and a radio function have emerged in a field of capsule medical apparatus. The capsule endoscopes are configured to move inside organs (body cavity) such as the stomach and small intestine accompanying peristaltic movements thereof to successively capture images using the imaging function in an observation period after being swallowed by a patient, who is a subject, for observation (examination) until being naturally discharged from a living body (human body) of the patient.

Image data captured inside the body cavity by the capsule endoscopes during the observation period of movements through these organs is sequentially transmitted to an external apparatus provided outside the subject by the radio function such as radio communication before being stored in memory provided in the external apparatus. If a patient carries an external apparatus equipped with the radio and memory functions, the patient can move freely without any inconvenience in an observation period until a capsule endoscope is discharged after swallowing the capsule endoscope. After the observation period, a diagnosis can be made based on image data stored in memory of the external apparatus by a physician or a nurse by causing a display means such as a display device to display images inside the body cavity.

A swallow-type capsule endoscope such as disclosed by Patent Document 1 has been known as this type of capsule endoscope, which proposes a configuration in which a reed switch turned on/off by an external magnetic field to control driving of the capsule endoscope is internally provided and the capsule endoscope is housed as a package including a permanent magnet for supplying the external magnetic field. That is, the reed switch provided in the capsule endoscope has a structure that maintains an off state under an environment in which a magnetic field of a certain strength or stronger is given, and sets an on state as the strength of the external magnetic field decreases. Thus, a capsule endoscope does not operate while the capsule endoscope is housed in a package. By taking out the capsule endoscope from the package before being swallowed, driving starts when the capsule endoscope ceases to be influenced by a magnetic force by being moved away from the permanent magnet. With the configuration described above, a capsule endoscope while packaged can be prevented from being driven and, after being taken out from the package, the capsule endoscope captures images using the imaging function of the capsule endoscope and transmits image signals by the radio function.

Patent Document 1: International Publication WO01/35813 Pamphlet

### DISCLOSURE OF INVENTION

### PROBLEM TO BE SOLVED BY THE INVENTION

However, the capsule endoscope normally involves a plurality of processes such as swallowing the capsule endoscope together with a fluid into a body of a subject and, for examination of stomach, drinking a plurality of fluids with different specific gravities and swallowing a blowing agent. These processes must be carried out in a specific order, but since Patent Document 1 does not consider supplying ingesta materials necessary for examination such as a capsule endoscope and fluids to a patient based on the specific order, it has been difficult in some cases for a patient to easily take in these ingesta materials in a specific order when, for example, ingesta materials increase in number.

WO 2005/060348 A2 discloses an in vivo device for imaging a body lumen. The in vivo device has a specific gravity of about 1 or a volume to weight ratio that enables it to float. The in vivo device includes an image sensor, which is attached to one or more buoyant bodies.

WO 2005/117801 A1 discloses a medical liquid container having a plurality of partitioning chambers and medical liquid-housing chambers, which communicate with one another, and a discharge reserve chamber having formed therein a discharge port for a liquid medicament. The medical liquid container further comprises a liquid tight partitioning portion for separating the liquid medicament-housing chambers from each other and a non-liquid tight partitioning portion for separating at least one of said liquid medicament-housing chambers and the discharge reserve chambers.

The present invention, which is defined in independent claim 1 and its dependent claims, has been developed in view of the above circumstances and an object thereof is to provide a capsule housing apparatus that allows a patient to take in accurately and easily a plurality of ingesta materials required for examination in a specific order.

### MEANS FOR SOLVING PROBLEM

A capsule housing apparatus according to an aspect of the present invention includes a capsule medical apparatus to be introduced into a subject; ingesta materials to be taken by the subject, the ingesta materials including at least one of drinkable fluid and a blowing agent for expanding a luminal organ of the subject; and a housing unit having a plurality of housing areas to house the capsule medical apparatus and at least one of the ingesta materials in each of the housing areas to make the capsule medical apparatus and the ingesta materials suppliable to the subject in a specific order.

In the capsule housing apparatus, the fluid may be a cleaning fluid or water.

In the capsule housing apparatus, the fluid may contain at least two fluids having at least two different temperatures, and the at least two fluids are suppliable to the subject in the specific order.

In the capsule housing apparatus, the fluid may contain at least two fluids having at least two different concentrations, and the at least two fluids are suppliable to the subject in the specific order.

In the capsule housing apparatus, the fluid may contain at least two fluids having at least two different specific gravities, and the at least two fluids are suppliable to the subject in the specific order.

In the capsule housing apparatus, the housing unit houses the capsule medical apparatus and the fluid in the same housing area.

In the capsule housing apparatus, the housing unit may divide the at least one fluid and houses the fluid in a plurality of housing areas for each of the fluids to make each fluid housed by dividing among the plurality of housing areas suppliable to the subject in the specific order in accordance with an intake amount necessary for the subject.

In the capsule housing apparatus, the capsule medical apparatus may further include a first magnetic material guidable from outside the subject, and the housing unit may further include a housing area for housing a second magnetic material for guiding the capsule medical apparatus by a magnetic interaction with the first magnetic material.

In the capsule housing apparatus, the first magnetic material may be one of a magnetic material having no magnetic pole, a permanent magnet, and an electromagnet.

In the capsule housing apparatus, the second magnetic material may be one of a permanent magnet and an electromagnet.

In the capsule housing apparatus, the housing unit may further include a housing area for housing a template for instructing a position of the second magnetic material outside the subject.

In the capsule housing apparatus, the housing unit further includes a housing area for housing a remover for removing mucus and foams inside the luminal organ.

In the capsule housing apparatus, the housing unit houses the fluid and the remover for removing mucus and foams inside the luminal organ in the same housing area.

The capsule housing apparatus may further include a partition wall that separates each of the housing areas and allows penetration between the housing areas in the specific order under a predetermined pressure.

In the capsule housing apparatus, a marking denoting the specific order may be marked to each of the housing areas.

In the capsule housing apparatus, the marking denoting the specific order may be a number denoting a specific order.

The capsule housing apparatus may further include a state detector that detects a state of the capsule medical apparatus, wherein timing for supplying the capsule medical apparatus and the ingesta materials to the subject in the specific order is determined in accordance with a detection result by the state detector.

In the capsule housing apparatus, the state detector may be a position detector that detects a position of the capsule medical apparatus.

A supplying method not according to the present invention uses a capsule housing apparatus, the capsule housing apparatus having a capsule medical apparatus to be introduced into a subject, a drinkable fluid used for examination of the subject, a blowing agent used for the examination of the subject to expand a luminal organ inside the subject, and a housing unit that has a plurality of housing areas, houses the capsule medical apparatus and at least one of the fluid and the blowing agent in each of the housing areas, and makes suppliable to the subject from each of the housing areas in a specific order. The supplying method includes the steps of causing the housing areas of the housing unit to open in the specific order; and making the subject to take in order of the fluid and the capsule medical apparatus in the opened housing areas, in order of the capsule medical apparatus and the fluid, in order of the capsule medical apparatus and the blowing agent, in order of the fluid, the capsule medical apparatus, and the blowing agent, in order of the fluid, the blowing agent, and the capsule medical apparatus, in order of the blowing agent, the fluid, and the capsule medical apparatus, in order of the capsule medical apparatus, the fluid, and the blowing agent, or in order of the capsule medical apparatus, the blowing agent, and the fluid.

A supplying method not according to the present invention uses a capsule housing apparatus, the capsule housing apparatus having a capsule medical apparatus to be introduced into a subject, a drinkable fluid used for examination of the subject, a blowing agent used for the examination of the subject to expand a luminal organ inside the subject, and a housing unit that has a plurality of housing areas, houses the capsule medical apparatus and at least one of the fluid and the blowing agent in each of the housing areas, and makes suppliable to the subject in order of markings denoting the specific order on each of the housing areas. The supplying method includes the steps of causing the housing areas of the housing unit to open in order of markings; and making the subject to take from the opened housing areas in order of the fluid and the capsule medical apparatus, in order of the capsule medical apparatus and the fluid, in order of the capsule medical apparatus and the blowing agent, in order of the fluid, the capsule medical apparatus, and the blowing agent, in order of the fluid, the blowing agent, and the capsule medical apparatus, in order of the blowing agent, the fluid, and the capsule medical apparatus, in order of the capsule medical apparatus, the fluid, and the blowing agent, or in order of the capsule medical apparatus, the blowing agent, and the fluid.

In the supplying method, the capsule medical apparatus may include a first magnetic material guidable from outside the subject, the housing unit may further include a housing area for housing a second magnetic material for guiding the capsule medical apparatus, and the steps may include supplying from the opened housing areas in order of the fluid, the capsule medical apparatus having the first magnetic material, and the second magnetic material, in order of the capsule medical apparatus having the first magnetic material, the blowing agent, and the second magnetic material, in order of the fluid, the capsule endoscope having the first magnetic material, the blowing agent, and the second magnetic material, or in order of the capsule medical apparatus having the first magnetic material, the blowing agent, the fluid, and the second magnetic material to the subject.

In the supplying method, the housing unit may further include a housing area for housing a template instructing a position of the second magnetic material and the steps may supply from the opened housing areas in order of the template, the fluid, the capsule medical apparatus having the first magnetic material, and the second magnetic material, in order of the template, the capsule medical apparatus having the first magnetic material, the blowing agent, and the second magnetic material, in order of the template, the fluid, the capsule medical apparatus having the first magnetic material, the blowing agent, and the second magnetic material, or in order of the template, the capsule medical apparatus having the first magnetic material, the blowing agent, the fluid, and the second magnetic material to the subject.

In the supplying method, the housing unit may further include a housing area for housing a remover for removing mucus and foams inside the luminal organ and the steps may cause the subject to take from the opened housing areas in order of the remover, the fluid, and the capsule medical apparatus, in order of the remover, the capsule medical apparatus, and the blowing agent, in order of the remover, the fluid, the capsule medical apparatus, and the blowing agent, or in order of the remover, the capsule medical apparatus, the blowing agent, and the fluid.

In the supplying method, the housing unit may further include a housing area for housing a remover for removing mucus and foams inside the luminal organ and the steps supply from the opened housing areas in order of the remover, the fluid, the capsule medical apparatus having the first magnetic material, and the second magnetic material, in order of the remover, the capsule medical apparatus having the first magnetic material, the blowing agent, and the second magnetic material, in order of the remover, the fluid, the capsule medical apparatus having the first magnetic material, the blowing agent, and the second magnetic material, or in order of the remover, the capsule medical apparatus having the first magnetic material, the blowing agent, the fluid, and the second magnetic material to the subject.

In the supplying method, the housing unit may further include a housing area for housing a remover for removing mucus and foams inside the luminal organ and the steps supply from the opened housing areas in order of the template, the remover, the fluid, the capsule medical apparatus having the first magnetic material, and the second magnetic material, in order of the template, the remover, the capsule medical apparatus having the first magnetic material, the blowing agent, and the second magnetic material, in order of the template, the remover, the fluid, the capsule medical apparatus having the first magnetic material, the blowing agent, and the second magnetic material, or in order of the template, the remover, the capsule medical apparatus having the first magnetic material, the blowing agent, the fluid, and the second magnetic material to the subject.

A supplying method not according to the present invention uses a capsule housing apparatus, the capsule housing apparatus having a capsule medical apparatus to be introduced into a subject, at least two drinkable fluids used for examination of the subject, and a housing unit that has a plurality of housing areas, houses the capsule medical apparatus and at least one of the at least two fluids in each of the housing areas, and makes suppliable to the subject from each of the housing areas in a specific order. The supplying method includes the steps of causing the housing areas of the housing unit to open in the specific order; and making the subject to take in order of a first fluid of the fluids, the capsule medical apparatus, and a second fluid other than the first fluid from the opened housing areas, in order of the first fluid of the fluids, the second fluid other than the first fluid, and the capsule medical apparatus, or in order of the capsule medical apparatus, the first fluid of the fluids, and the second fluid other than the first fluid.

In the supplying method, the housing unit may further include a housing area housing a blowing agent for expanding a luminal organ of the subject, and the steps may cause the subject to take in order of the first fluid of the fluids, the capsule medical apparatus, the blowing agent, and the second fluid other than the first fluid from the opened housing areas, in order of the first fluid of the fluids, the blowing agent, the capsule medical apparatus, and the second fluid other than the first fluid, in order of the first fluid of the fluids, the second fluid other than the first fluid, the blowing agent, and the capsule medical apparatus, or in order of the first fluid of the fluids, the capsule medical apparatus, the second fluid other than the first fluid, and the blowing agent.

A supplying method not according to the present invention uses a capsule housing apparatus, the capsule housing apparatus having a capsule medical apparatus to be introduced into a subject, at least two drinkable fluids used for examination of the subject, a blowing agent used for the examination of the subject to expand a luminal organ inside the subject, and a housing unit that has a plurality of housing areas, houses the capsule medical apparatus and at least one of each of the fluids and the blowing agent in each of the housing areas, and makes suppliable to the subject in order of markings denoting the specific order on each of the housing areas. The supplying method includes the steps of causing the housing areas of the housing unit to open in order of markings; and making the subject to take in order of a first fluid of the fluids, the capsule medical apparatus, the blowing agent, and a second fluid other than the first fluid from the opened housing areas, in order of the first fluid of the fluids, the blowing agent, the capsule medical apparatus, and the second fluid other than the first fluid, in order of the first fluid of the fluids, the second fluid other than the first fluid, the blowing agent, and the capsule medical apparatus, or in order of the first fluid of the fluids, the capsule medical apparatus, the second fluid other than the first fluid, and the blowing agent.

In the supplying method, the capsule medical apparatus may include a first magnetic material guidable from outside the subject, the housing unit may further include a housing area for housing a second magnetic material for guiding the capsule medical apparatus, and the steps may include supplying in order of the first fluid of the fluids, the capsule medical apparatus having the first magnetic material, the blowing agent, the second fluid other than the first fluid, and the second magnetic material from the opened housing areas, or in order of the capsule medical apparatus having the first magnetic material, the blowing agent, the first fluid of the fluids, the second fluid other than the first fluid, and the second magnetic material to the subject.

In the supplying method, the housing unit may further include a housing area for housing a template instructing a position of the second magnetic material, and the steps may include supplying in order of the template, the first fluid of the fluids, the capsule medical apparatus having the first magnetic material, the blowing agent, the second fluid other than the first fluid, and the second magnetic material from the opened housing areas, or in order of the template, the capsule medical apparatus having the first magnetic material, the blowing agent, the first fluid of the fluids, the second fluid other than the first fluid, and the second magnetic material to the subject.

In the supplying method, the housing unit may further include a housing area for housing a remover for removing mucus and foams inside the luminal organ, and the steps may cause the subject to take in order of the remover, the first fluid of the fluids, the capsule medical apparatus, the blowing agent, and the second fluid other than the first fluid from the opened housing areas, or in order of the remover, the capsule medical apparatus, the blowing agent, the first fluid of the fluids, and the second fluid other than the first fluid.

In the supplying method, the housing unit may further include a housing area for housing a remover for removing mucus and foams inside the luminal organ, and the steps may include supplying in order of the remover, the first fluid of the fluids, the capsule medical apparatus having the first magnetic material, the blowing agent, the second fluid other than the first fluid, and the second magnetic material from the opened housing areas, or in order of the remover, the capsule medical apparatus having the first magnetic material, the blowing agent, the first fluid of the fluids, the second fluid other than the first fluid, and the second magnetic material to the subject.

In the supplying method, the housing unit may further include a housing area for housing a remover for removing mucus and foams inside the luminal organ and the steps may supply in order of the template, the remover, the first fluid of the fluids, the capsule medical apparatus having the first magnetic material, the blowing agent, the second fluid other than the first fluid, and the second magnetic material from the opened housing areas, or in order of the template, the remover, the capsule medical apparatus having the first magnetic material, the blowing agent, the first fluid of the fluids, the second fluid other than the first fluid, and the second magnetic material to the subject.

A supplying method not according to the present invention uses a capsule medical system, the capsule medical system having a capsule medical apparatus to be introduced into a subject, a drinkable fluid used for examination of the subject, and a blowing agent used for the examination of the subject to expand a luminal organ inside the subject. The supplying method includes the step of causing the subject to take in order of the fluid and the capsule medical apparatus, in order of the capsule medical apparatus and the fluid, in order of the capsule medical apparatus and the blowing agent, in order of the fluid, the capsule medical apparatus, and the blowing agent, in order of the fluid, the blowing agent, and the capsule medical apparatus, in order of the blowing agent, the fluid, and the capsule medical apparatus, in order of the capsule medical apparatus, the fluid, and the blowing agent, or in order of the capsule medical apparatus, the blowing agent, and the fluid.

A supplying method not according to the present invention uses a capsule medical system, the capsule medical system having a capsule medical apparatus to be introduced into a subject and at least two drinkable fluids used for examination of the subject. The supplying method includes the step of causing the subject to take in order of a first fluid of the fluids, the capsule medical apparatus, and a second fluid other than the first fluid, in order of the first fluid of the fluids, the second fluid other than the first fluid, and the capsule medical apparatus, or in order of the capsule medical apparatus, the first fluid of the fluids, and the second fluid other than the first fluid.

In the supplying method, the capsule medical system may further include a blowing agent used for examination of the subject to expand a luminal organ of the subject, and the steps may cause the subject to take in order of the first fluid of the fluids, the capsule medical apparatus, the blowing agent, and the second fluid other than the first fluid, in order of the first fluid of the fluids, the blowing agent, the capsule medical apparatus, and the second fluid other than the first fluid, in order of the first fluid of the fluids, the second fluid other than the first fluid, the blowing agent, and the capsule medical apparatus, or in order of the first fluid of the fluids, the capsule medical apparatus, the second fluid other than the first fluid, and the blowing agent.

A supplying method not according to the present invention uses a capsule medical system, the capsule medical system having a capsule medical apparatus to be introduced into a subject and at least one ingesta material that is used for examination of the subject and necessary for the subject to take in. The supplying method includes a first supply step of supplying the capsule medical apparatus; a detection step of detecting a state of the capsule medical apparatus supplied by the first supply step; an intake timing determination step of determining intake timing of the ingesta material in accordance with a detection result of the state of the capsule medical apparatus; and a second supply step of supplying one of the ingesta material to the subject in the intake timing determined by the intake timing determination step.

In the supplying method, the detection step may detect a position of the capsule medical apparatus.

In the supplying method, the detection step and the second supply step may be repeated as many times as a number of ingesta materials to be taken in by the subject.

The supplying method may further include, between the intake timing determination step and the second supply step, a postural change timing determination step of determining postural change timing of the subject in accordance with a detection result of a capsule medical apparatus state; and a postural change step of changing a posture of the subject in the postural change timing determined in the postural change timing determination step.

### EFFECT OF THE INVENTION

A capsule housing apparatus according to the present invention provides a configuration that houses a capsule medical apparatus and at least one of the fluid and blowing agent in a plurality of housing areas of a housing unit and allows supplies in a specific order, and therefore, an effect of making the patient capable of taking in accurately and easily a plurality of ingesta materials required for examination in a specific order is brought about. The invention is defined in independent claim 1 and the dependent claims.

### BRIEF DESCRIPTION OF DRAWINGS

FIG. 1 is a schematic view showing an overall configuration of a wireless in-vivo information acquiring apparatus including a capsule medical apparatus according to the present invention;
FIG. 2 is a perspective view showing the configuration of a capsule housing apparatus according to a first embodiment for housing ingesta material to be taken by a patient;
FIG. 3 is a schematic view showing a state in which the patient takes in the ingesta material shown in FIG. 2;
FIG. 4 is an outline flow chart showing a procedure for a method of supplying the ingesta material;
FIG. 5 is an outline front view showing how it looks like when making a stomach observation according to the first embodiment;
FIG. 6 is an outline front view showing how it looks like inside the stomach before and after increasing an intake of a first fluid;
FIG. 7 is a perspective view showing the configuration of a first modification of the capsule housing apparatus according to the first embodiment shown in FIG. 2;
FIG. 8 is similarly a perspective view showing the configuration of a second modification of the capsule housing apparatus according to the first embodiment;
FIG. 9 is a perspective view showing the configuration of a capsule housing apparatus according to a second embodiment;
FIG. 10 is a partial enlarged view enlarged at a part of FIG. 9;
FIG. 11 is an outline front view showing how it looks like when making a stomach observation according to the second embodiment;
FIG. 12 is a front view showing the configuration of a modification of the capsule housing apparatus according to the second embodiment shown in FIG. 9;
FIG. 13 is a perspective view showing the configuration of a capsule housing apparatus according to a third embodiment;
FIG. 14 is a block diagram showing an internal configuration of a drive control system of the capsule housing apparatus shown in FIG. 13;
FIG. 15 is a flow chart for describing operations of the drive control system;
FIG. 16 is a perspective view showing the configuration of a first modification of the capsule housing apparatus according to the third embodiment shown in FIG. 13;
FIG. 17 is similarly a perspective view showing the configuration of a second modification of the capsule housing apparatus according to the third embodiment; and
FIG. 18 is an outline flow chart showing another procedure for the method of supplying ingesta materials using the capsule housing apparatus.

### EXPLANATIONS OF LETTERS OR NUMERALS

1 Subject (patient)
2 Receiving device
2a Antenna unit
2b Main receiving unit
3 Capsule endoscope
4 Display device
5 Portable recording medium
6, 30, 50 Capsule housing apparatus
7 First fluid (liquid)
8 Second fluid
10, 20, 40 Package
11, 12, 21, 22, 21a to 21c, 22a, 22b, 31a to 31c, 41, 42 Housing area
13, 25a, 25b, 26, 43 Partition wall
14, 23, 24, 44 Swallowing mouth
31 Blister pack
31d Sealing surface
32 Cylindrical bottle
32a Graduation
33 Housing case
35, 35a, 35b, 35c Sterilized sheet
36 Straw
51 Outer frame body
52 Covering device
53 Supply button
54 Fluid discharger
55 Solid material output port
56 Display unit
57 Weight sensor
58 Control unit
59 Fluid discharge actuator
60 Solid material output actuator
61 Cup
62, 70 ID reader
63, 65 ID information
64 Arm
71, 72 Wireless ID tag
A1 to An Receiving antenna

### BEST MODES FOR CARRYING OUT THE INVENTION

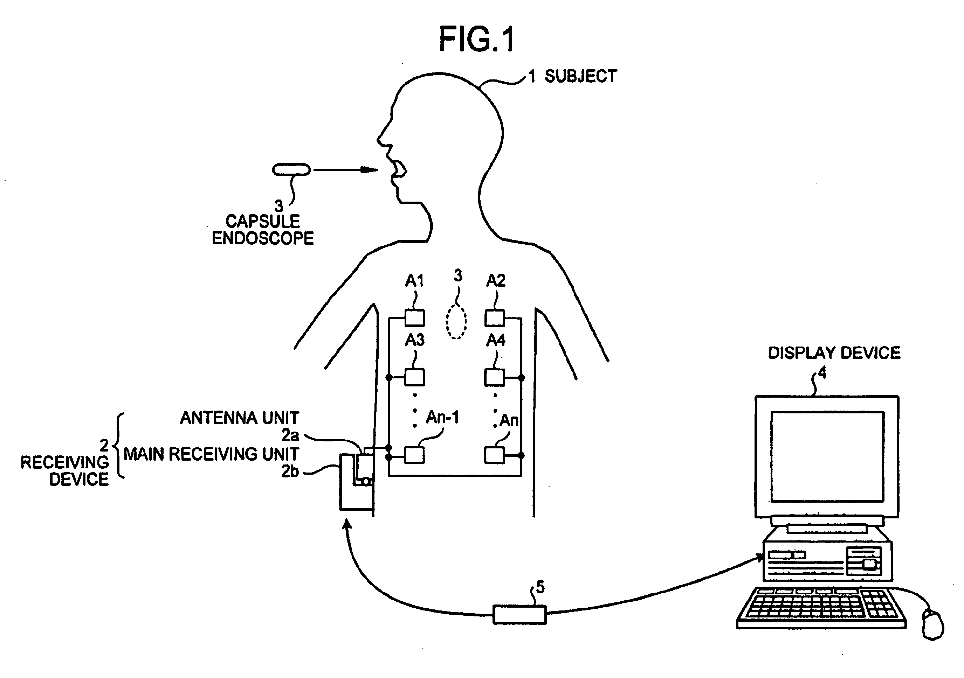

Embodiments of a capsule housing apparatus according to the present invention and a method of supplying the capsule medical apparatus using a capsule housing apparatus will be described in detail below with reference to drawings of FIG. 1 to FIG. 18. However, the present invention is not limited to the these embodiments and can be carried out in various modifications without departing from the scope of the present invention.

### First embodiment

FIG. 1 is a schematic view showing an overall configuration of a wireless in-vivo information acquiring apparatus including a capsule medical apparatus according to the present invention. In the wireless in-vivo information acquiring apparatus (also called a capsule medical system), a capsule endoscope for capturing images of regions to be examined in a body cavity by being introduced into the body cavity from a mouth of a human, who is a subject, will be described as an example of the capsule medical apparatus. In FIG. 1, the wireless in-vivo information acquiring apparatus comprises a receiving device 2 having a radio receiving function and a capsule endoscope 3 that is introduced into a subject 1 and transmits data such as image signals to the receiving device 2 after capturing images inside a body cavity. The wireless in-vivo information acquiring apparatus also comprises a display device 4 for displaying an image inside the body cavity based on an image signal received by the receiving device 2 and a portable recording medium 5 for exchanging data between the receiving device 2 and the display device 4.

The receiving device 2 comprises an antenna unit 2a having a plurality of receiving antennas A1 to An attached to an extracorporeal surface of the subject 1 and a main receiving unit 2b for performing processing of radio signals received via the plurality of receiving antennas A1 to An, and these units are detachably connected via a connector or the like. Each of the receiving antennas A1 to An may be, for example, attached to a jacket wearable by the subject 1 so that the subject 1 is furnished with the receiving antennas A1 to An by wearing the jacket. In this case, the receiving antennas A1 to An may be detachable from the jacket.

The display device 4 is used to display images such as images inside the body cavity captured by the capsule endoscope 3 and has a configuration such as a workstation that displays images based on data obtained from the portable recording medium 5. More specifically, the display device 4 may be configured to directly display images such as a CRT display and a liquid crystal display, or to output images to another medium such as a printer.

The portable recording medium 5 has a structure in which the portable recording medium 5 is detachable from the main receiving unit 2b and the display device 4 and, when inserted into both, can output or record information. In the first embodiment, the portable recording medium 5 is inserted into the main receiving unit 2b to record data transmitted from the capsule endoscope 3 while the capsule endoscope 3 moves inside the body cavity of the subject 1. Then, after the capsule endoscope 3 is discharged from the subject 1, that is, capturing of images inside the subject 1 is completed, the portable recording medium 5 is ejected from the main receiving unit 2b and inserted into the display device 4 so that data recorded in the portable recording medium 5 is read by the display device 4. For example, by exchanging data between the main receiving unit 2b and the display device 4 using the portable recording medium 5 constructed of Compact Flash (registered trademark) memory, the subject 1 can move more freely while capturing images inside the body cavity than when the main receiving unit 2b and the display device 4 are directly connected by a wire. Meanwhile, the portable recording medium 5 is used here for exchanging data between the main receiving unit 2b and the display device 4, but the present embodiment is not limited to this and, for example, the main receiving unit 2b may be configured to use another built-in recording device such as harddisk and to connect the recording device and the display device 4 by cable communication or radio communication to exchange data between the recording device and the display device 4.

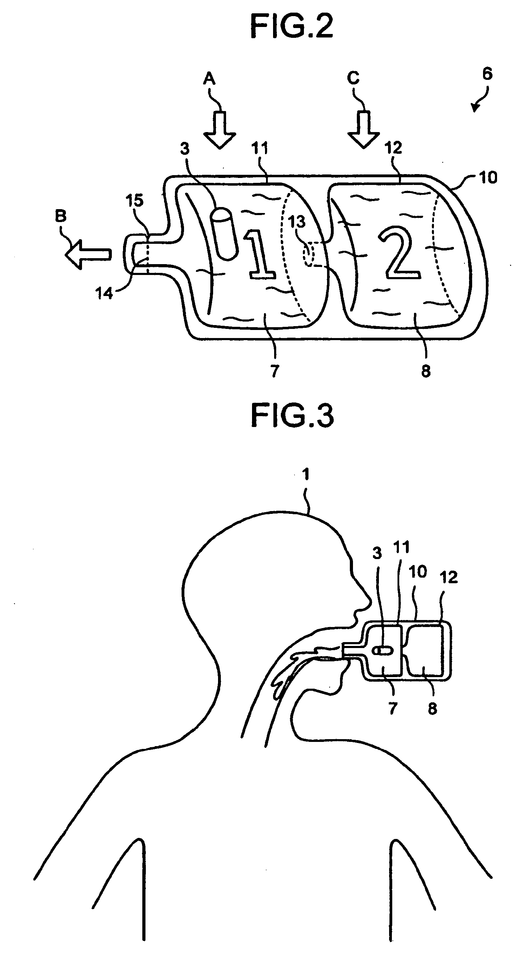

Meanwhile, the capsule endoscope 3 needs to be sterilized and a sterilized state must be maintained before using the capsule endoscope 3 for a patient. In some examinations using the capsule endoscope 3, in addition to the capsule endoscope 3, a plurality of ingesta materials such as a fluid (a fluid for elongating or expanding a luminal organ or a cleaning fluid for cleaning a lumen) and a blowing agent for elongating the luminal organ must be administered to a patient in specific order. Thus, in the first embodiment, a plurality of ingesta materials such as the sterilized capsule endoscope 3 are housed in a capsule housing apparatus. FIG. 2 is a perspective view showing the configuration of a capsule housing apparatus 6 according to the first embodiment for housing ingesta materials to be taken by a patient.

First, the capsule housing apparatus 6 in FIG. 2 comprises a package 10 as a housing unit having housing areas 11 and 12 that house the capsule endoscope 3 and a fluid, and a partition wall 13 provided between the housing areas 11 and 12 to penetrably separate each of the housing areas 11 and 12. The package 10 is formed, for example, of resin material like an approximately cylindrical bag, and has internally the two-partitioned housing areas 11 and 12.

The housing areas 11 and 12 are formed in an approximately cylindrical shape. The housing area 11 houses, for example, the capsule endoscope 3 and a first fluid used for the subject (patient) 1 to swallow the capsule endoscope 3, and the housing area 12 houses a second fluid 8. The partition wall 13 separating the housing areas 11 and 12 is disposed between the housing areas 11 and 12. The partition wall 13 operates to separate the housing areas 11 and 12 in response to a pressure from a direction of the housing area 11 (a pressure applied from the direction of the housing area 11) and operates to allow penetration of the housing areas 11 and 12 in response to a pressure from the direction of the housing area 12 (a pressure applied from the direction of the housing area 12) in the same manner as, for example, a check valve. Or, the partition wall 13 operates to break in response to a certain level of pressure or higher from the direction of the housing area 12. Meanwhile, such housing areas are not limited to two areas and the first and second fluids 7 and 8 can be finely divided and housed by providing a plurality of housing areas in the package 10.

A swallowing mouth 14 is provided at one end of the housing area 11 to cause ingesta materials in the housing areas 11 and 12 to discharge. The swallowing mouth 14 is formed to normally block the housing area 11 from the outside and is constructed in such a way that the patient 1 can cut off part of the swallowing mouth 14 before swallowing the capsule endoscope 3 to cause the housing area 11 and the outside to communicate. A slit 15 can also be provided in the package 10 near the swallowing mouth 14 so that part of the swallowing mouth 14 can easily be cut. Further, numbers "1" and "2" are marked on the package 10 over the housing areas 11 and 12 to notify the patient of the order in which ingesta materials should be swallowed.

The first fluid 7 and the second fluid 8 may be of different liquid quality. For example, the first fluid 7 may be water whose specific gravity is 1, while the second fluid 8 may be a fluid with a different specific gravity composed of food oil (such as olive oil) whose specific gravity is smaller than 1. If the first fluid 7 and the second fluid 8 have such different liquid quality, for example, they may be fluids that can be drunken by the patient 1 to control a position of the capsule endoscope 3 in a luminal organ to be examined. The first fluid 7 and the second fluid 8 may also be of the same liquid quality. For example, the first fluid 7 and the second fluid 8 may be fluids of the same specific gravity. If the first fluid 7 and the second fluid 8 have the same liquid quality, for example, they may be fluids that can be drunken by the patient 1 when an amount of fluids to be swallowed is controlled in accordance with physical constitution of the patient 1. For the capsule endoscope 3, the specific gravity of the fluid 7 and that of the fluid 8 may be related as (first fluid 7) > (capsule endoscope 3) > (second fluid 8), or (first fluid 7) = (second fluid 8) > (capsule endoscope 3).

The capsule endoscope 3 is, for example, a one-eye type including an imaging optical system capable of capturing images in a front end direction, circuit systems such as a board, circuit components, and a transmitting antenna, a battery, an acceleration sensor, and an angular velocity sensor inside a capsule casing (not shown) insertable into a body cavity of the subject 1, and is formed by sealing an inner part of the capsule casing fluid-tightly. The capsule endoscope 3 captures images of objects (organ interiors of the subject 1) positioned in a gravitational direction while drifting in a fluid. The capsule endoscope 3 as described above may be formed, for example, in such a way that a front end side is relatively light to change a balance of a center of gravity in a fore-and-aft direction so that images in an upward gravitational direction can be captured, or in such a way that a front end side is relatively heavy to change the balance of the center of gravity in the fore-and-aft direction so that images in a downward gravitational direction can be captured.

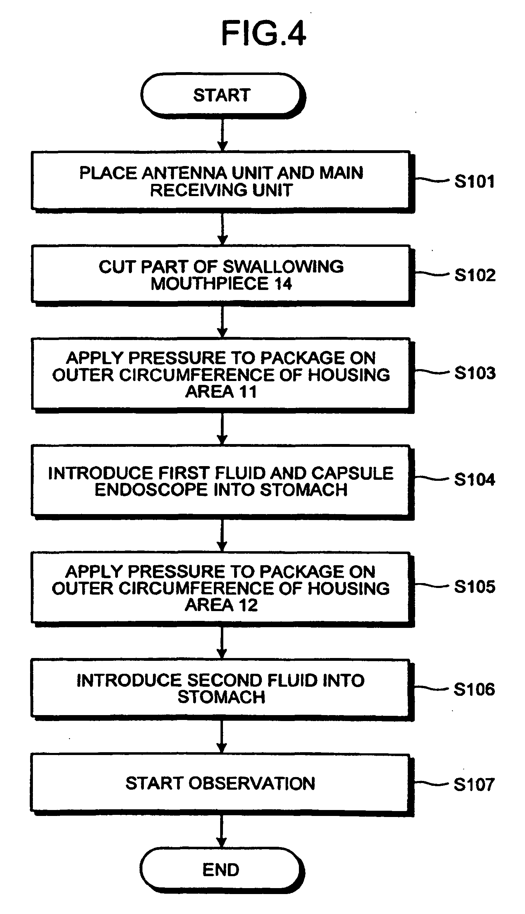

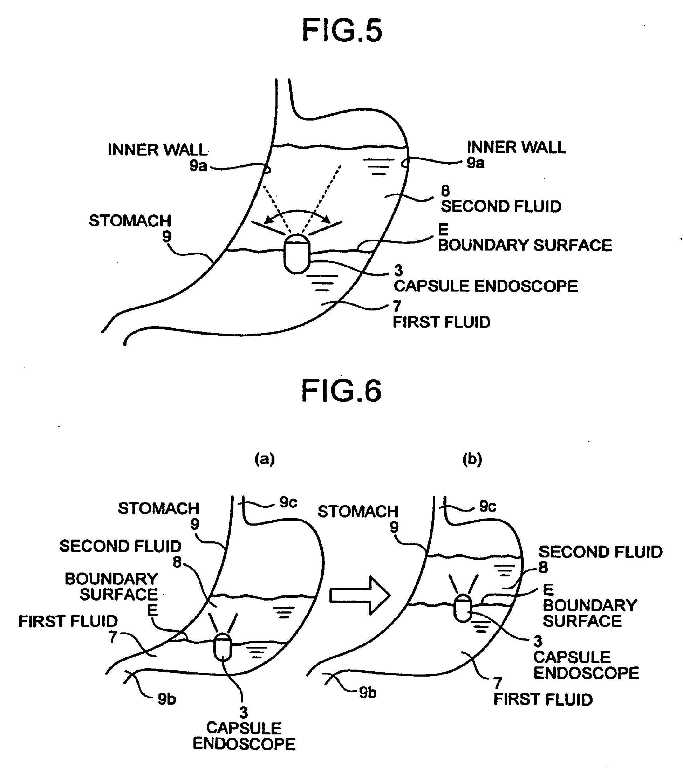

A series of methods of supplying the capsule endoscope 3 when the capsule endoscope 3 is taken in by the patient 1 using the capsule housing apparatus 6 before being introduced, for example, into the stomach will be described with reference to drawings in FIG. 3 to FIG. 5. FIG. 3 is a schematic view showing a state in which a patient takes in the ingesta material shown in FIG. 2, FIG. 4 is an outline flow chart showing a procedure for a method of supplying the ingesta material, and FIG. 5 is an outline front view showing how it looks like when making a stomach observation. For a description of the first embodiment below, it is assumed that the capsule endoscope 3, the specific gravity of the fluid 7 and that of the fluid 8 are related by (first fluid 7) > (capsule endoscope 3) > (second fluid 8), and the balance of the center of gravity in the fore-and-aft direction is changed by making a front end side of the capsule endoscope 3 relatively light so that, for example, images can be captured by the imaging optical system in the upward gravitational direction while drifting in a fluid in the gravitational direction before being introduced into a stomach 9.

In FIG. 4, before supplying ingesta material, first the antenna unit 2a having the receiving antennas A1 to An for receiving signals from the capsule endoscope 3 is placed at a predetermined position of the subject 1 and the main receiving unit 2b is placed near the subject 1 (step S101). Next, part of the swallowing mouth 14 is cut (step S102), the swallowing mouth 14 is brought closer to the mouth, as shown in FIG. 3, and the capsule endoscope 3 and the fluid 7 in the housing area 11 are discharged into the mouth (arrow B direction in FIG. 2) of the patient by applying a pressure (pressure in an arrow A direction in FIG. 2) to the package 10 on an outer circumference of the housing area 11 (for example, a partial area of the package 10 where the swallowing order "1" is marked) with a finger (step S103).

The patient 1 can thereby swallow the first fluid 7 and the capsule endoscope 3 into the body and the swallowed first fluid 7 and the capsule endoscope 3 are introduced into the stomach 9 (step S104). In this case, the patient 1 swallows ingesta material in a standing position or a sitting position. In this state, the partition wall 13 in the package 10 maintains a closed state to separate the housing area 11 and the housing area 12. The capsule endoscope 3 may be activated, like a conventional example, by mounting a permanent magnet on the package 10 and removing the capsule endoscope 3, or bringing a permanent magnet closer to the capsule endoscope 3 after removing the capsule endoscope 3 from the package 10. Since LED of the imaging optical system of the capsule endoscope 3 flashes after activation, it is preferable to construct a package from transparent material so that the patient can check the flashing.

Next, by applying a pressure (pressure in an arrow C direction in FIG. 2) to the package 10 on the outer circumference of the housing area 12 (for example, a partial area of the package 10 where the swallowing order "2" is marked) (step S105), the partition wall 13 opens to the housing area 11 to allow penetration from the housing area 12 to the housing area 11 so that the second fluid 8 in the housing area 12 is discharged into the mouth of the patient (arrow B in FIG. 2) via the housing area 11. The patient 1 can thereby swallow the second fluid 8 into the body and the swallowed second fluid 8 is introduced into the stomach 9 (step S106). After the capsule endoscope 3, the first fluid 7, and the second fluid 8 are introduced into the stomach 9, the stomach 9 expands based on an intake thereof and due to differences in specific gravity, as shown in FIG. 5, the second fluid 8 forms a boundary surface E on the first fluid 7 to form a layered state and the capsule endoscope 3 having an intermediate specific gravity drifts at a position of the boundary surface E. Since the balance of the center of gravity is changed in the fore-and-aft direction of the capsule endoscope 3 so that the front end is relatively light, the capsule endoscope 3 stabilizes in a standing state (vertical state) in which the front end, which is an imaging direction, is directed upward in the gravitational direction at the boundary surface E and drifts. The capsule endoscope 3 obtains images of an inner wall 9a by capturing images of an upper part of the stomach 9 in such a stable standing state and transmits the obtained images to the receiving device 2. As a result, observation of the stomach 9 can start (step S107).

Since, in the first embodiment, the capsule endoscope 3 and first fluid 7, and the second fluid 8 are housed in the plurality of the housing areas 11 and 12 of the package 10 respectively as ingesta materials for a subject, these housing areas 11 and 12 are made to communicate and also a partition wall is provided between the housing areas 11 and 12 in such a way that the partition wall is open by a pressure in one direction so that ingesta materials will be discharged, and ingesta materials can be supplied to the subject in a specific order based on numbers marked on the package 10, it is for a patient to accurately and easily take in a plurality of ingesta materials required for examination in a specific order. Since, in the first embodiment, the order of intake is made accurate, anyone can handle the package easily and the examination is simplified. Thus, probabilities of a patient taking in ingesta materials in incorrect order will thereby be reduced and examination will be carried out more smoothly, leading to prevention of incorrect examination and efficient examination time. Also, since the capsule housing apparatus 6 has only one package, no person other than the patient needs to touch the package so that examination can be completed hygienically.

Moreover, since the stomach 9 can be expanded by swallowing a fluid in the first embodiment, sufficient space inside the organ necessary for observation by the capsule endoscope 3 can be secured, more detailed images of the inner wall of the organ can be captured, and the stomach 9 can be observed without missing any part inside the stomach 9. The capsule endoscope 3 can be rocked randomly to drift at the boundary surface E only by changing the position of the boundary surface E inside the stomach 9 in combination with postural change of the patient 1 himself (herself). Random rocking of the capsule endoscope 3 can cause changes of imaging regions inside the stomach 9 to be captured by the capsule endoscope 3 and further allow for observation without missing any part inside the stomach 9. Furthermore, by using an imaging optical system with wider angles denoted by solid lines instead of dotted lines in FIG. 5 as the imaging optical system of the capsule endoscope 3, observations inside the stomach 9 can be made in still wider ranges with smaller postural changes.

Also in the first embodiment, the capsule endoscope 3 can be caused to make observations inside the stomach 9 at any drifting position of the capsule endoscope 3 in the gravitational direction inside the stomach 9 by changing a height position of the boundary surface E with variable intakes into the stomach 9 of the first fluid 7 and the second fluid 8. FIG. 6 is an outline front view showing how it looks like inside the stomach 9 before and after increasing an intake of the first fluid 7. That is, after starting observations by swallowing predetermined amounts of the first fluid 7 and second fluid 8 together with the capsule endoscope 3, as shown in FIG. 6 (a), the intake of the first fluid 7 inside the stomach 9 is sequentially increased by additionally taking in the first fluid 7 suitably to gradually raise the position of the boundary surface E, as shown in FIG. 6 (b), so that the inner wall 9a can be observed successively from a lower part (pyloric part of stomach) 9b of the stomach 9 to an upper part (cardiac part of stomach) 9c. Also in this case, imaging regions by the capsule endoscope 3 can be changed by changing only the position of the boundary surface E inside the stomach 9 in combination with postural change of the patient 1 himself (herself) each time the first fluid 7 is added, and thus images inside the stomach 9 can exhaustively captured without missing any part inside the stomach 9. If the front end of the capsule endoscope 3 is made relatively heavy to change the balance of the center of gravity in the fore-and-aft direction so that images in a downward gravitational direction can be captured by the imaging optical system, like the above case, imaging regions by the capsule endoscope 3 can be changed by changing only the position of the boundary surface E inside the stomach 9, allowing for exhaustive image capturing without missing any part inside the stomach 9. This makes it possible, after examination, to combine a plurality of images captured randomly based on correlations of each image to create an overall image inside the stomach 9 for presentation to a physician, leading to efficient diagnosis.

If fluids of the same specific gravity are used as the first fluid 7 and the second fluid 8, for example, water of the specific gravity 1 is used for both, effects similar to those described above can be brought about, but in this case, the capsule endoscope 3 is in a standing state at the boundary surface between water and air space. If the boundary surface fluctuates, since viscosity at the boundary surface is weaker than when two fluids have different specific gravities, the capsule endoscope 3 moves (falls) wildly and it becomes difficult for the capsule endoscope 3 to drift in a stable standing state with less movement. If two fluids having different specific gravities are used, since an observation plane is always in the fluids, cracks and stains on the observation plane of the capsule endoscope 3 are less noticeable and good observation images can be obtained. Further, since the stomach is filled with the first and second fluids in the first embodiment, supersonic waves propagate well. Thus, when capturing images, a three-dimensional image inside the stomach is captured by a 3D supersonic probe or the like provided outside the subject to determine the position and orientation of the capsule endoscope. Then, by combining images based on this position or orientation information, images of the stomach inner wall can be constructed with high precision and also a contribution to a high-precision diagnosis can be made.

The position of the capsule endoscope 3 can be detected, for example, by an acceleration sensor or an angular velocity sensor provided inside the capsule endoscope 3, but the receiving device 2 has a plurality of receiving antennas A1 to An for receiving radio signals from the capsule endoscope 3 and a position detection function to deduce the position of the capsule endoscope 3 from receiving strengths of each of the plurality of receiving antennas A1 to An may be provided. Then, the images may be combined based on a result of the position detection function. In this case, precision of the image combination is further improved so that more accurate diagnosis can be made.

### Modifications of the first embodiment

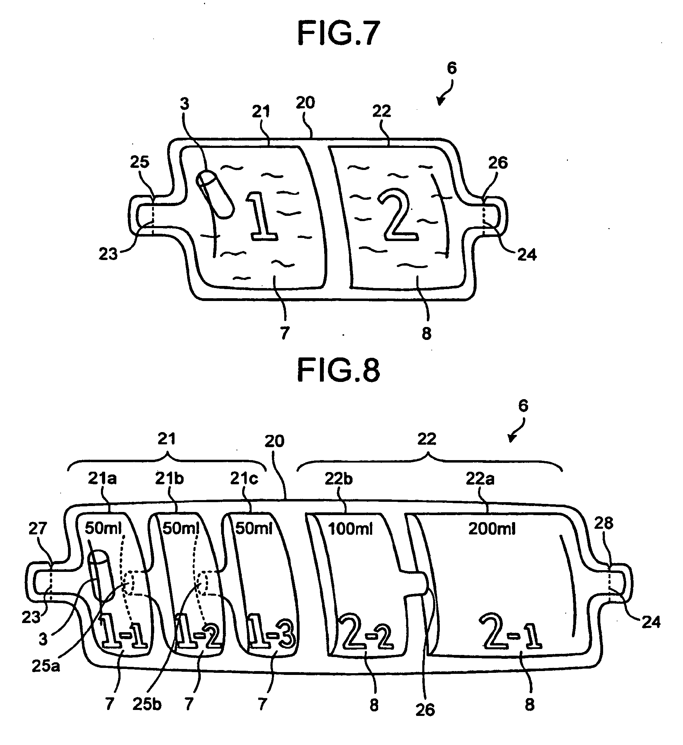

FIG. 7 is a perspective view showing the configuration of a first modification of the capsule housing apparatus 6 according to the first embodiment shown in FIG. 2. In FIG. 7, the capsule housing apparatus 6 has a package 20 having internally housing areas 21 and 22 that are completely separated under conditions of no penetrability. The package 20 has separate swallowing mouths 23 and 24 for the patient 1 to swallow ingesta material inside the housing areas 21 and 22 respectively provided at both ends.

The first modification of the first embodiment is similar to the first embodiment in that the capsule endoscope 3 and the first fluid are housed in the housing area 21 and the second fluid is housed in the housing area 22, and numbers "1" and "2" to notify the patient 1 of the order of swallowing are marked on the package 20 over the housing areas 21 and 22 respectively. Similarly, slits 25 and 26 can be provided in the package 20 near the swallowing mouths 23 and 24 so that part of the swallowing mouths 23 and 24 can easily be cut, like the first embodiment.

In the first modification of the first embodiment, the patient first cuts part of the swallowing mouth 23 and applies a pressure to the package 20 on the outer circumference of the housing area 21 (for example, a partial area of the package 20 where the swallowing order "1" is marked) to discharge the capsule endoscope 3 and the first fluid 7 inside the housing area 21 into the mouth of the patient 1. Subsequently, the patient 1 reverses the package 20 to bring the housing area 22 closer to the patient 1, and cuts part of the swallowing mouth 24 and applies a pressure to the package 20 on the outer circumference of the housing area 22 (for example, a partial area of the package 20 where the swallowing order "2" is marked) to discharge the second fluid 8 inside the housing area 22 into the mouth of the patient 1.

Since, in the first modification of the first embodiment, the capsule endoscope 3 and first fluid 7, and the second fluid 8 are also housed in the plurality of the housing areas 21 and 22 of the package 20 respectively as ingesta materials for the subject 1 and ingesta materials can be supplied to the subject 1 in a specific order based on numbers marked on the package 20, like the first embodiment, it is for the patient to accurately and easily take in a plurality of ingesta materials required for examination in a specific order. Meanwhile, marking on the package is not limited to the numbers and may be alphabetical characters such as "A", "B", and "C", or any other desired marking that allows presentation of a specific order may be used.

Since the capsule housing apparatus 6 according to the first modification of the first embodiment consists of the package 20 having the two housing areas 21 and 22, the structure thereof is simple and automation of production can easily be realized, facilitating production. Also, since the capsule housing apparatus 6 according to the first modification of the first embodiment has only one package like the first embodiment, no person other than the patient needs to touch the package so that examination can be completed hygienically.

FIG. 8 is a perspective view showing the configuration of a second modification of the capsule housing apparatus 6 according to the first embodiment shown in FIG. 2. In the second modification of the first embodiment, for example, the capsule housing apparatus 6 is appropriate when the first and second fluids of different liquid quality are taken in and the position of the capsule endoscope 3 is controlled inside a luminal organ (stomach) or the intake of fluids is changed in accordance with physical constitution of the patient 1 or the size of stomach. That is, in FIG. 8, the capsule housing apparatus 6 according to the second modification of the first embodiment comprises a housing area 21 including a plurality of housing areas 21a to 21c to house the first fluid 7 by dividing the first fluid 7 among the housing areas 21a to 21c and a housing area 22 including a plurality of housing areas 22a and 22b to house the second fluid 8 by dividing the second fluid 8 among the housing areas 22a and 22b. The housing area 21 and housing area 22 described above are completely separated under conditions of no penetrability.

A partition wall 25a that penetrably separates each of the housing areas 21a and 21b is provided between the housing areas 21a and 21b, and a partition wall 25b that penetrably separates each of the housing areas 21b and 21c is provided between the housing areas 21b and 21c. Between the housing areas 22a and 22b, on the other hand, a partition wall 26 that penetrably separates each of the housing areas 22a and 22b is provided. The partition walls 25a and 25b operate, like the partition wall 13 in the first embodiment, to allow penetration of the housing areas only in response to a pressure in the direction from the housing area 21c or housing area 21b to the housing area 21a in the same manner as, for example, a check valve. The partition wall 26 operates, like the partition wall 13 in the first embodiment, to allow penetration of the housing areas only in response to a pressure in the direction from the housing area 22b to the housing area 22a in the same manner as, for example, a check valve. The housing areas 21 and 22 are formed as completely separated areas and are not affected by any pressure applied to each other.

A swallowing mouth 23 for discharging the ingesta materials (the capsule endoscope 3 and the first fluid 7) in the housing areas 21a to 21c and a swallowing mouth 24 for discharging the ingesta material (the second fluid 8) in the housing areas 22a and 22b are provided at ends of the housing areas 21a and 22a respectively. The swallowing mouths 23 and 24 are formed to normally block the housing areas 21a and 22a from the outside respectively and are constructed so that the patient 1 can cut off part of the swallowing mouth 23 or 24 before swallowing the ingesta material to cause the housing area 21a or 22a and the outside to communicate respectively. Slits 27 and 28 can also be provided in the package 20 near the swallowing mouths 23 and 24 so that part of the swallowing mouths 23 and 24 can easily be cut. Further, numbers "1-1", "1-2", and "1-3" to notify the patient 1 of the swallowing order are marked on the package 20 over these housing areas 21a to 21c. On the package 20 over the housing areas 22a and 22b, on the other hand, numbers "2-1" and "2-2" to similarly notify the patient 1 of the swallowing order are marked. In the second modification of the first embodiment, for example, 50 ml of the first fluid 7 is housed in each of the housing areas 21a to 21c, and the capsule endoscope 3 is housed in the housing area 21a together with the first fluid 7. 200 ml of the second fluid 8 is housed in the housing area 22a and 100 ml of the second fluid 8 is housed in the housing area 22b. The second fluid 8 is divided among the housing areas 22a and 22b to be housed there. For example, 200 ml of the second fluid 8 is housed in the housing area 22a and 100 ml of the second fluid 8 is housed in the housing area 22b. In the second modification of the first embodiment, the capsule endoscope 3 can also be housed in a housing area separately from the first fluid 7.

When the patient 1 takes in the capsule endoscope 3 using the capsule housing apparatus 6 described above, part of the swallowing mouth 23 is first cut off and a pressure is applied to a housing area (one of the housing areas 21a to 21c) of a predetermined required intake. To take in 100 ml of the first fluid 7 and the capsule endoscope 3, for example, the capsule endoscope 3 and the first fluid 7 (100 ml) inside the housing areas 21a and 21b can be discharged into the mouth (arrow B direction in FIG. 2) of the patient 1 by applying a pressure to the package 20 on the outer circumference of the housing area 21b (for example, a partial area of the package 20 where the swallowing order "1-2" is marked) with a finger. The patient 1 can thereby swallow the capsule endoscope 3 and a required amount of the first fluid 7 into the body. In this state, the partition wall 25a is open to the housing area 21a to allow penetration between the housing area 21 and housing area 21b. The partition wall 25b, on the other hand, remains closed to separate the housing area 21a and the housing area 21b and prevents the first fluid 7 in the housing area 21c from discharging from the housing area 21c. Since the patient can take in a fluid by directly putting the swallowing mouth 23 into the mouth in this form, the fluid can easily be taken in regardless of the posture of the patient. If, for example, the patient is in a left decubitus, a right decubitus, or a supine posture, the patient can still take in the fluid easily.

Next, the patient 1 reverses the package 20 to bring the housing area 22 closer to the patient 1, and cuts part of the swallowing mouth 24 and applies a pressure to a housing area (either of the housing areas 22a and 22b) of a predetermined required intake. To take in 200 ml of the second fluid 8, for example, the second fluid 8 (200 ml) inside the housing area 22a can be discharged into the mouth of the patient 1 by applying a pressure to the package 20 on the outer circumference of the housing area 22a (for example, a partial area of the package 20 where the swallowing order "2-1" is marked). The patient 1 can thereby swallow a required amount of the second fluid 8 into the body. In this state, the partition wall 26 remains closed to separate the housing area 22a and the housing area 22b and prevents the second fluid 8 in the housing area 22b from discharging from the housing area 22b.

Since, in the second modification of the first embodiment as described above, the capsule endoscope 3 and first fluid 7, and the second fluid 8 are also housed in the plurality of the housing areas 21 and 22 of the package 20 respectively as ingesta materials for the subject 1 and ingesta materials can be supplied to the subject 1 in a specific order based on numbers marked on the package 20, the same effects as those of the first embodiment can be brought about. Also, since the first and second fluids 7 and 8 are divided among the housing areas 21a to 21c and the housing areas 22a and 22b to be housed there before a required amount of intake can be discharged to the outside, suitable amounts of ingesta material can be supplied to the patient as required. For a patient of an average physical constitution, a total required intake of about 500 ml (supplied, for example, by dividing 500 ml into three housing areas of 100 ml, 200 ml, and 200 ml) is sufficient. For a patient of still larger build, it is more preferable to construct the capsule housing apparatus 6 having housing areas capable of housing ingesta materials of up to about 1000 ml and to use the capsule housing apparatus 6 to make ingesta materials such as fluids of up to about 1000 ml suppliable to the patient.

### Second embodiment

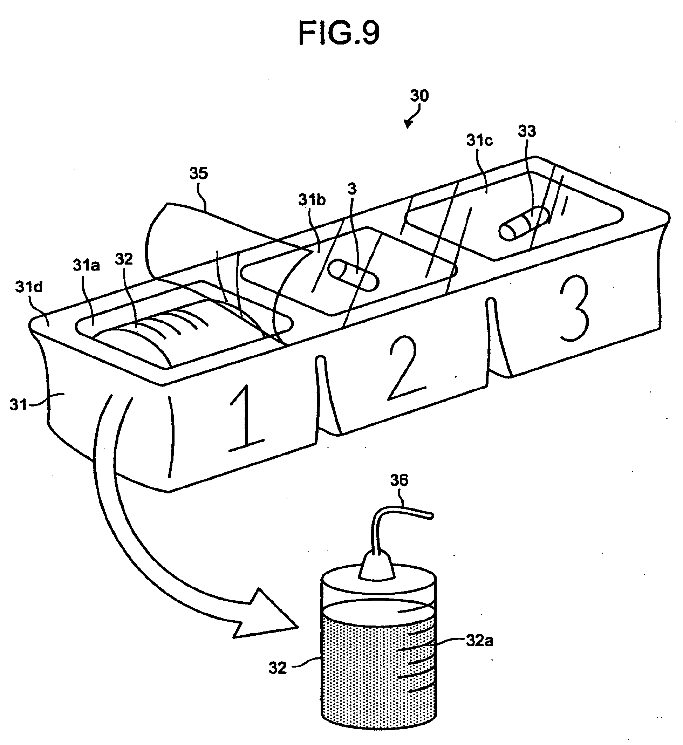

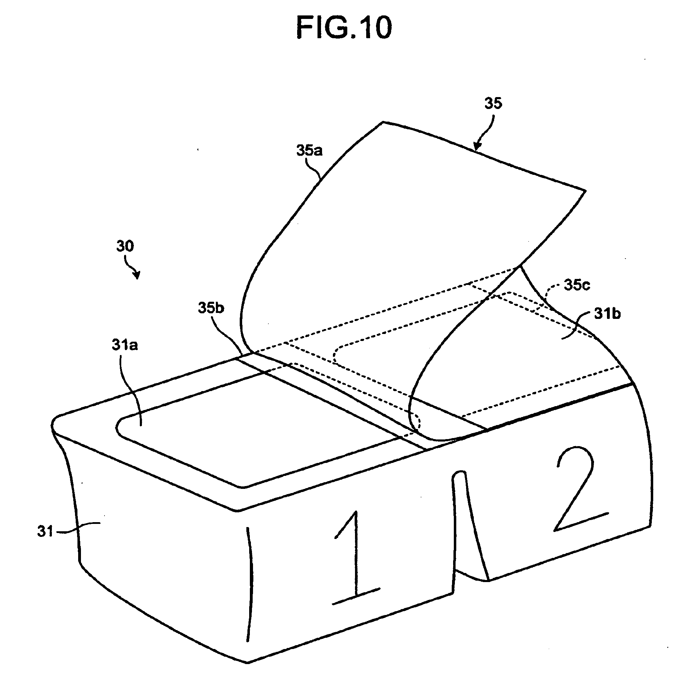

FIG. 9 is a perspective view showing the configuration of a capsule housing apparatus according to a second embodiment for housing ingesta material to be taken by a patient, and FIG. 10 is a partial enlarged view that enlarges part of FIG. 9. In FIG. 9 and FIG. 10, a capsule housing apparatus 30 comprises a blister pack 31 as a housing unit having housing areas 31a to 31c for housing a fluid, the capsule endoscope 3, and a blowing agent respectively, and a sterilized sheet 35 provided on a top surface of an opening of the blister pack 31. The blister pack 31 has the housing areas 31a to 31c formed like three boxes with a concave cross section, and is formed by coupling the top surface of opening of these boxes lined up in a row. On the top surface of the opening, a sealing surface 31d for blocking up the opening using the sterilized sheet 35 by heat sealing is provided. The sealing surface 31d is formed by surrounding in an endless form the outer circumference of the top surface of the opening of the housing areas 31a to 31c disposed in a row.

A fluid is housed inside the housing area 31a and "1" to notify the patient 1 of the swallowing order is marked on the side of the blister pack 31 where the housing area 31a is formed. The fluid consists of water of specific gravity 1 and is housed in the housing area 31a, for example, in a state in which the fluid is housed in a cylindrical bottle 32 having a straw 36 inserted through an upper part thereof and graduations 32a by which a total volume of the fluid and an intake of the patient are recognizable. The capsule endoscope 3 having the same configuration as that in the first embodiment with a specific gravity set to a little less than 1 is housed in the housing area 31b, and "2" to similarly make the swallowing order known is marked on the side of the blister pack 31 where the housing area 31b is formed. A blowing agent for expanding the stomach of the patient 1 is housed in the housing area 31c, and "3" to similarly make the swallowing order known is marked on the side of the blister pack 31 where the housing area 31c is formed. The blowing agent is housed in the housing area 31c, for example, in a state in which the blowing agent is housed in a housing case 33.

The sterilized sheet 35 is sealed a little longer than the sealing surface 31d, for example, on edges of the housing area 31a and one sheet of the sterilized sheet 35 blocks up the top surface of the opening of the blister pack 31. The sterilized sheet 35 may be made tearable from the housing area 31a by holding a ligulate part sticking out of the sterilized sheet 35 described above to tear off the sterilized sheet 35 from the blister pack 31. Also, the sterilized sheet 35 may be provided as three sterilized sheets 35a, 35b, and 35c corresponding to the three housing areas 31a, 31b, and 31c and, as shown in an enlarged view in FIG. 10 for example, parts of the sterilized sheets 35a and 35b of the adjacent housing areas may be overlapped to block up the top surface of each opening. In this case, the top surface of the opening of the sterilized sheet 35c of the housing area 31c whose swallowing order of the patient 1 is last is first blocked up and the top surface of the opening of the sterilized sheet 35a of the housing area 31a whose swallowing order is first is lastly blocked up. Edges of the sterilized sheets in later order are sealed to cover the housing area of the previous order a little. In this case, it is preferable to make adhesive strength between each sterilized sheet and the sealing surface 31d stronger than that between sterilized sheets to prevent each sterilized sheet from being removed one after another.

Thus, as shown in FIG. 10, when the sterilized sheet 35a of the housing area 31a over which the number "1" is marked is removed from the blister pack 31, the bottle 32 in which water is housed and an edge of the sterilized sheet 35b of the second housing area 31b appear and so that the sterilized sheet 35b of the next housing area 31b can be torn off. The patient 1 can take out the bottle 32 to swallow a required amount of fluid (for example, water) into the body. Since the bottle 32 has the graduations 32a showing the volume of water, the patient 1 drinks an amount of water in accordance with the size of body of the patient himself (herself) through the straw 36 while consulting the graduations 32a. The intake of water to be taken by the patient 1 may be determined based on information such as weight, height, and chest measurement of the patient 1 measured in advance, or based on information such as the size of stomach obtained in advance from X rays or abdominal echo.

Next, when the patient 1 removes the sterilized sheet 35b of the housing area 31b over which the number "2" is marked from the blister pack 31, the capsule endoscope 3 and an edge of the sterilized sheet 35c of the third housing area 31c appear and so that the sterilized sheet 35c of the next housing area 31c can be torn off. The patient 1 takes the capsule endoscope 3 in his (her) hand before swallowing the capsule endoscope 3. The patient 1 can thereby swallow water and the capsule endoscope 3 into the body and the swallowed water and capsule endoscope 3 are introduced into the stomach 9. The capsule endoscope 3 is activated in the same manner as that in the first embodiment.

Then, when the sterilized sheet 35c of the housing area 31c over which the number "3" is marked is lastly removed from the blister pack 31, the housing case 33 in which a blowing agent is housed appears. The patient 1 takes a blowing agent in his (her) hand before swallowing the blowing agent. When swallowing the blowing agent, water for causing the blowing agent to react (foam) may also be taken in. When a blowing agent is taken in, pressure inside the stomach rises and the patient wants to belch, but the belch must be suppressed for examination. Thus, after taking in a blowing agent, it is difficult to take in a fluid or a capsule endoscope. Consequently, in terms of this, the patient preferably takes in the blowing agent last. If observations should be made by dropping a capsule endoscope into the stomach expanded by a blowing agent or a fluid, the capsule endoscope (or another fluid) may naturally be swallowed after taking in the blowing agent as needed.

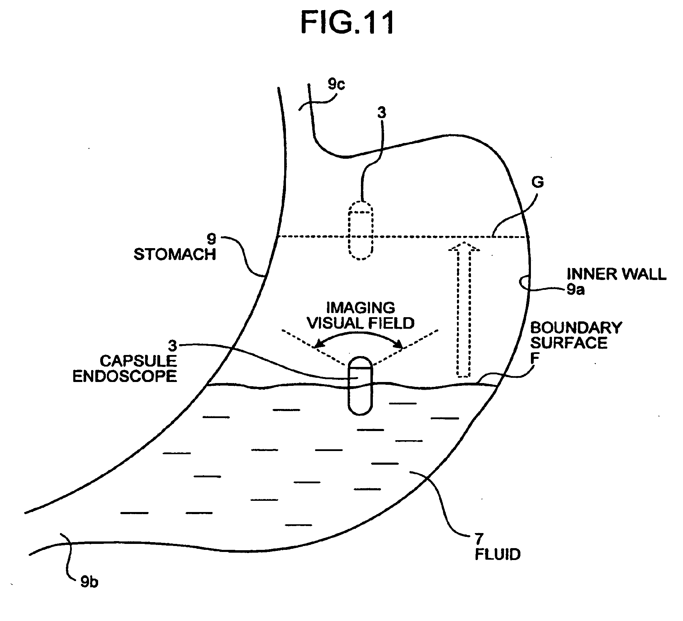

If the capsule endoscope 3, fluids, and a blowing agent are introduced into the stomach 9, the stomach 9 expands due to the blowing agent and, as shown in FIG. 11, a boundary surface F is formed by the fluid 7 and air space due to differences in specific gravity and the capsule endoscope 3 having a specific gravity smaller than that of the fluid 7 is positioned at the boundary surface F to drift there. Since the balance of the capsule endoscope 3 in the fore-and-aft direction is changed so that the front end thereof is relatively light, the capsule endoscope 3 stabilizes in a standing state (vertical state) in which the front end, which is an imaging direction, is directed upward in the gravitational direction at the boundary surface F and drifts. The capsule endoscope 3 obtains images of the inner wall 9a by capturing images of the upper part of the stomach 9 in such a stable standing state and transmits the obtained images to the receiving device 2. As a result, observation of the stomach 9 can start.

Also, in the second embodiment, the capsule endoscope 3 can be caused to make observations inside the stomach 9 at any drifting position of the capsule endoscope 3 in the gravitational direction inside the stomach 9 by changing the height position of the boundary surface F with a variable intake into the stomach 9 of the first fluid 7. That is, after starting observations by swallowing a predetermined amount of the fluid 7 together with the capsule endoscope 3, the intake of the fluid 7 inside the stomach 9 is sequentially increased by additionally taking in the fluid 7 suitably to gradually raise the position of the boundary surface F, for example from a solid line position to a dotted line position G, so that the inner wall 9a can be observed successively from the lower part (pyloric part of stomach) 9b of the stomach 9 to the upper part (cardiac part of stomach) 9c. Also in this case, imaging regions by the capsule endoscope 3 can be changed by changing only the position of the boundary surface F inside the stomach 9 in combination with postural change of the patient 1 himself (herself) each time the fluid 7 is added, and thus images inside the stomach 9 can exhaustively captured without missing any part inside the stomach 9. If the front end of the capsule endoscope 3 is made relatively heavy to change the balance in the fore-and-aft direction so that images in the downward gravitational direction can be captured by the imaging optical system, like the above case, imaging regions by the capsule endoscope 3 can be changed by changing only the position of the boundary surface F inside the stomach 9, allowing for exhaustive image capturing without missing any part inside the stomach 9. This makes it possible, after examination, to combine a plurality of images captured randomly based on correlations of each image to create an overall image inside the stomach 9 for presentation to a physician, leading to efficient diagnosis.

Since, in the second embodiment, a plurality of ingesta materials such as a fluid, the capsule endoscope 3, and a blowing agent are housed in the partitioned housing areas 31a to 31c of the blister pack 31 respectively, the sterilized sheet 35 is removed in a specific order based on the numbers marked on the blister pack 31, and each ingesta material is made suppliable to the subject in the specific order, it is for a patient to accurately and easily take in a plurality of ingesta materials required for examination in a specific order. Since some types of capsule-shaped capsule medical apparatuses are dissolved in fluids, like the second embodiment, deformation and deterioration of individual ingesta materials can be prevented by housing each ingesta material in a separate housing area.

Some types of body cavity examination using the capsule endoscope 3 do not require an intake of a fluid or a blowing agent. In such a case, it is possible to make a patient to take in the capsule endoscope 3 and a blowing agent in this order or a fluid and the capsule endoscope 3 in this order by housing the capsule endoscope 3 and blowing agent or the fluid and capsule endoscope 3 in separate housing areas of the blister pack 31 and removing the sterilized sheets 35 in a specific order based on numbers marked on the blister pack 31. Also, in some cases, water is necessary to take in a blowing agent; therefore, it is possible to house the water in the housing area of the blister pack 31 and, considering the above case, to allow the patient to take in the capsule endoscope 3, the blowing agent, and the water in this order. It is also possible to house only a fluid and a blowing agent or a fluid and part of a blowing agent in housing areas of the blister pack 31 because of storage or transport characteristics.

If the capsule endoscope 3 contains a first magnetic material (a permanent magnet) that can be guided from outside a subject, the capsule endoscope 3 can be guided such as moving in a horizontal direction or rocking at the spot by applying a magnetic field to the capsule endoscope 3 from outside the subject using a magnetic material (a permanent magnet or an electromagnet). If, here, the magnetic material outside the subject used for guidance is a permanent magnet, a new housing area is formed in a backmost part of the blister pack 31 and the permanent magnet is housed in the housing area like other ingesta materials to clarify the order of using the permanent magnet. Then, it is also possible to make a patient to take or obtain, for example, in order of a fluid, the capsule endoscope 3, and a permanent magnet, in order of the capsule endoscope 3, a blowing agent, and a permanent magnet, in order of a fluid, the capsule endoscope 3, a blowing agent, and a magnet, or in order of the capsule endoscope 3, a blowing agent, water, and a permanent magnet. In this case, it is more preferable to construct the blister pack 31 from a.magnetic closed circuit that blocks a magnetic field of any housed magnet.

Further, if the position of a magnet outside a subject is instructed using a template, a new housing area for housing the template is formed as the first one in the blister pack 31 and the template is housed in the housing area like other ingesta materials. Then, it is also possible to make a patient to take or obtain, for example, in order of a template, a fluid, the capsule endoscope 3, and a permanent magnet, in order of a template, the capsule endoscope 3, a blowing agent, and a permanent magnet, in order of a template, a fluid, the capsule endoscope 3, a blowing agent, and a magnet, or in order of a template, the capsule endoscope 3, a blowing agent, water, and a permanent magnet.

A patient may also take a remover (such as Gascon, dimethylpolysiloxane, pronase, proctase, and sodium acid carbonate) for removing mucus inside the stomach, foams by a blowing agent, or mucus itself before taking in a fluid. In this case, a new housing area for housing a remover may be formed as the first one in the blister pack 31, or the remover may be mixed with a fluid housed in a housing area. Then, it is also possible to make a patient to take, for example, in order of a remover, a fluid, and the capsule endoscope 3, in order of a remover, the capsule endoscope 3, and a blowing agent, in order of a remover, a fluid, the capsule endoscope 3, and a blowing agent, or in order of a remover, the capsule endoscope 3, a blowing agent, and a fluid. In addition, an antispasmodic (such as a peppermint solution, oxethazaine, scopolia extract, and timepidium bromide) may be supplied in a specific order so that a patient can take in the antispasmodic.

### Modification of the second embodiment

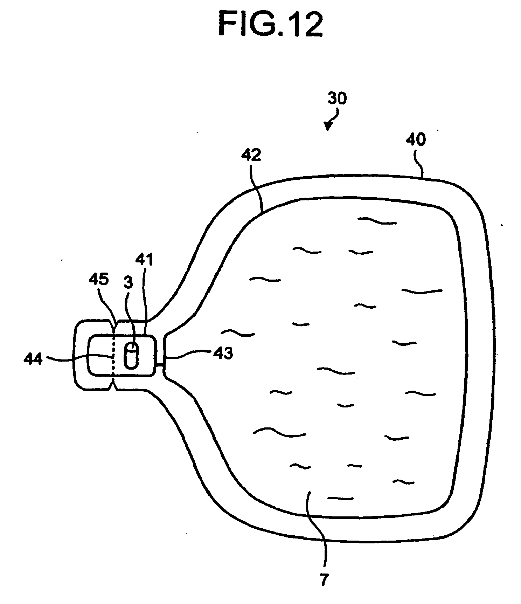

FIG. 12 is a front view showing the configuration of a modification of the capsule housing apparatus 30 according to the second embodiment shown in FIG. 9. In FIG. 12, the capsule housing apparatus 30 according to the present modification of the second embodiment comprises a package 40 as a housing unit having housing areas 41 and 42 housing the capsule endoscope 3 and the fluid 7 respectively and a partition wall 43 provided between the housing areas 41 and 42 to penetrably separate each of the housing areas 41 and 42. The package 40 is formed, for example, of resin material like an approximately cylindrical bag, and has internally the two-partitioned housing areas 41 and 42. Since the present modification of the second embodiment houses each of ingesta materials separately in each housing area, the present modification is classified as a modification of the second embodiment.

The housing areas 41 and 42 are formed in an approximately cylindrical shape and house the capsule endoscope 3 and the fluid 7 separately. The housing area 41 houses, for example, the capsule endoscope 3 and the housing area 12 houses the fluid 7 used for the patient 1 to swallow the capsule endoscope 3. The partition wall 43 separating the housing areas 41 and 42 is disposed between the housing areas 41 and 42. The partition wall 43 operates to separate the housing areas 41 and 42 in response to a pressure from the direction of the housing area 41 (a pressure applied from the direction of the housing area 41) and operates to allow penetration of the housing areas 41 and 42 in response to a pressure from the direction of the housing area 42 (a pressure applied from the direction of the housing area 42) in the same manner as, for example, a check valve.

A swallowing mouth 44 is provided at one end of the housing area 41 to cause ingesta materials in the housing areas 41 and 42 to discharge. The swallowing mouth 44 is formed to normally block the housing area 41 from the outside and is constructed in such a way that the patient 1 can cut off part of the swallowing mouth 44 before swallowing the capsule endoscope 3 to cause the housing area 41 and the outside to communicate. A slit 45 can also be provided in the package 40 near the swallowing mouth 44 so that part of the swallowing mouth 44 can easily be cut.

In the present modification of the second embodiment, the patient first cuts off part of the swallowing mouth 44 to take out the capsule endoscope 3 from the housing area 41 and next applies a pressure to the package 40 on the outer circumference of the housing area 42 to discharge the fluid inside the housing area 42 into the mouth of the patient.

Since, in the present modification of the second embodiment as described above, the fluid and the capsule endoscope 3 are housed in separate housing areas and each ingesta material is made suppliable to the subject in a specific order by cutting off part of the swallowing mouth 44, like the second embodiment, it is for the patient to accurately and easily take in a plurality of ingesta materials required for examination in the specific order, and also to prevent deformation and deterioration of individual ingesta materials.

Meanwhile, the present modification of the second embodiment was described for a case in which the capsule endoscope 3 is housed, but instead of the capsule endoscope 3, a solid or powder blowing agent, for example, may be housed in the housing area 41. Since, in this case, the blowing agent deforms or deteriorates due to moisture content, deformation or deterioration of the blowing agent before being swallowed by the patient can be prevented by housing the blowing agent separately from the fluid in each housing area. Then, it is also possible to enable the patient to take in the capsule endoscope 3 and thereafter a blowing agent and water.

### Third embodiment

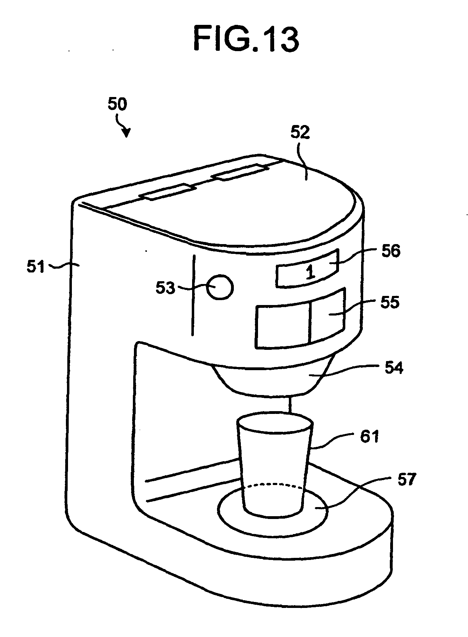

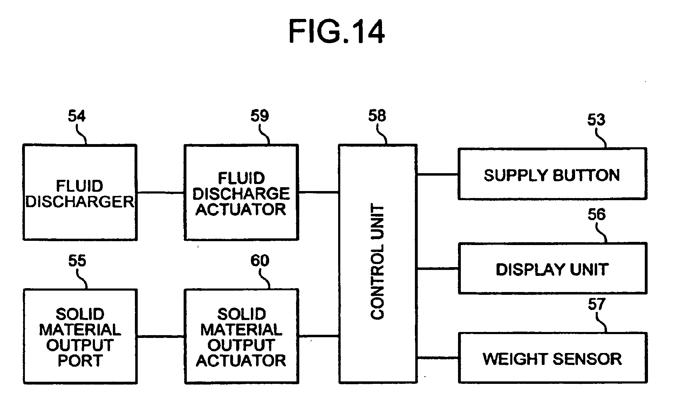

FIG. 13 is a perspective view showing the configuration of a capsule housing apparatus 50 according to a third embodiment housing ingesta material to be taken by a patient, and FIG. 14 is a block diagram showing an internal configuration of a drive control system of the capsule housing apparatus 50 shown in FIG. 13. In contrast to the packages and blister pack described above, in the third embodiment, a plurality of ingesta materials to be taken by a patient are supplied in order mechanically and actively. In FIG. 13 and FIG. 14, the capsule housing apparatus 50 comprises an outer frame body 51 as a housing unit whose side has a U shape, a covering device 52 for covering the top surface of opening of the outer frame body 51, a supply button 53 for instructing to supply ingesta material, a fluid discharger 54 as a housing area provided on the upper part of the outer frame body 51 to house and discharge first and second fluids, a solid material output port 55 as a housing area provided on the upper part of the outer frame body 51 to house and output solid materials such as the capsule endoscope 3 and a blowing agent among the capsule endoscope 3 and ingesta materials, a display unit 56 provided on the upper part of the outer frame body 51 to make known the order of examination and a specific order of taking in a plurality of ingesta materials, and a weight sensor 57 provided on the lower part of the outer frame body 51 to detect a container provided on the outer frame body 51, for example, a cup 61.

The fluid discharger 54 is disposed on an undersurface of the upper part of the outer frame body 51 and discharges the first and second fluids housed internally toward the weight sensor 57 below by drive control of a fluid discharge actuator 59. Meanwhile, the fluid discharger 54 is configured so that the first and second fluids are housed in separate housing areas and discharged separately based on a specific order.

The solid material output port 55 is disposed on the side of the upper part of the outer frame body 51 and causes the capsule endoscope 3 and a blowing agent housed internally to be carried out in a lateral direction of the outer frame body 51 by drive control of a solid material output actuator 60 so that the patient 1 can take out. The solid material output port 55 may be configured to carry out both the capsule endoscope 3 and a blowing agent simultaneously or separately. The ingesta material can be replenished by opening the covering device 52 covering the top surface of the opening of the outer frame body 51 to refill the fluid discharger 54 and solid material output port 55 from above.

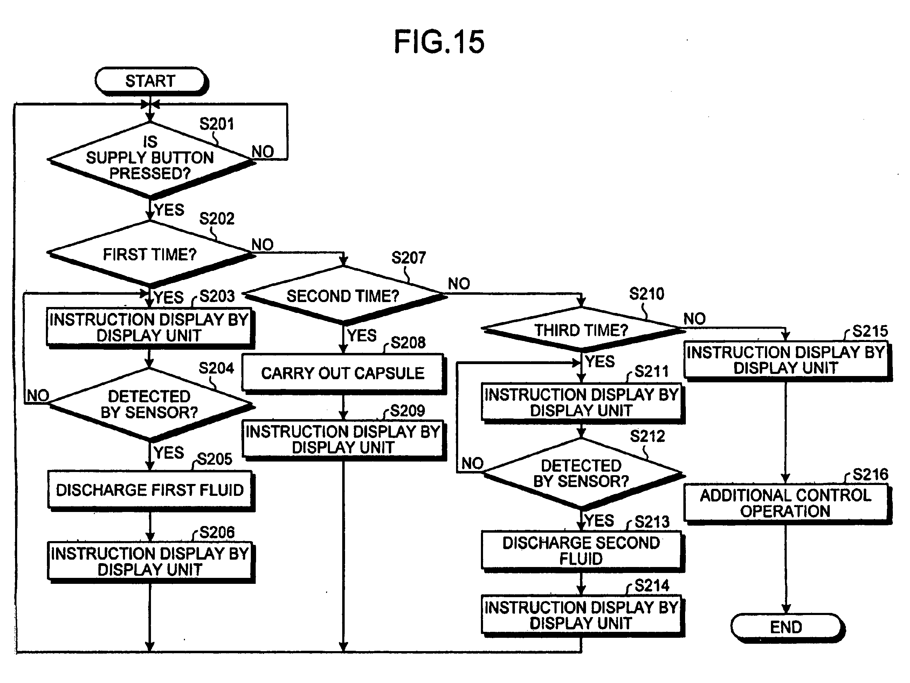

The display unit 56 consists, for example, of a display unit for performing liquid crystal display, and displays a step to be performed next by the patient in order when the supply button 53 is pressed. The weight sensor 57 detects the weight of the cup 61 put on the weight sensor 57 and, when the cup 61 reaches a fixed weight by inflow of a fluid, informs a control unit 58 of detection information that the fixed weight has been reached. The control unit 58 performs display and drive control of the display unit 56, fluid discharge actuator 59, and solid material output actuator 60 in accordance with instruction information from the supply button 53 and detection information from the weight sensor 57. Operations of the display and drive control by the control unit 58 will be described below using the flow chart in FIG. 15.

First, when the patient 1 to be examined presses the supply button 53 (steps S201, S202), the control unit 58 performs the display control of the display unit 56 to cause a screen of the display unit 56 to display "1" indicating a specific order and "Fluid will come out. Put a cup." (step S203). Then, after the cup 61 is put on the weight sensor 57, the weight sensor 57 detects that the cup 61 has been put on (step S204) and the control unit 58 performs the drive control of the fluid discharge actuator 59 to cause the fluid discharger 54 to discharge the first fluid in accordance with a detection signal from the weight sensor 57 (step S205). The capsule housing apparatus 50 has an input means of information and the control unit 58 determines types of fluid required for examination and intakes thereof based on patient information (such as the physical constitution or stomach size of the patient, or examination circumstances on that day) input into the input means in advance from outside or transferred via a hospital network to cause the fluid discharger 54 to supply the determined types of fluid and intakes.

When the discharged first fluid remains in the cup 61 and the weight sensor 57 detects a fixed weight, the control unit 58 causes the fluid discharger 54 to stop discharging the first fluid and the screen of the display unit 56 to display "2" indicating the specific order and "Please drink the fluid. Press the supply button after you have drunk the fluid." (step S206). The patient drinks the first fluid and then presses the supply button 53 according to the instruction (steps S201, S207).

If the supply button 53 is pressed again, the control unit 58 performs the drive control of the solid material output actuator 60 to cause the solid material output port 55 to carry the capsule endoscope 3 out of the capsule housing apparatus 50 (step S208). Then, the screen of the screen unit 56 is caused to display "3" indicating the specific order and "Please swallow the capsule. Press the supply button after you have drunk the capsule." (step S209). The capsule endoscope 3 is already activated when carried out by the solid material output port 55. The capsule housing apparatus 50 has an electromagnet for capsule activation in the solid material output port 55 and a magnetic switch inside the capsule endoscope 3 is turned on by causing the electromagnet to generate a magnetic field to activate the capsule endoscope 3.

Next, when the supply button 53 is pressed (steps S201, S210), the control unit 58 causes the screen of the display unit 56 to display "4" indicating the specific order and "Fluid will come out. Put a cup." (step S211). Then, when the cup 61 is put on the weight sensor 57 (step S212), the control unit 58 causes the fluid discharger 54 to discharge the second fluid in accordance with a detection signal from the weight sensor 57 (step S213). When a fixed amount of the second fluid gathers in the cup 61, the control unit 58 causes the fluid discharger 54 to stop discharging the second fluid and the screen of the display unit 56 to display "5" indicating the specific order and "Please drink the fluid." (step S214). The patient drinks the second fluid and then presses the supply button 53 according to the instruction (step S201).

Subsequently, the capsule housing apparatus 50 continues to issue examination instructions such as causing the patient to additionally drink the first fluid to control the drifting position of the capsule endoscope 3 inside the stomach. In this case, the control unit 58 causes the display unit 56 to display information about the examination instructions. That is, the examination instructions are issued by displaying, for example, "Please drink the first fluid." on the screen of the display unit 56 when the supply button 53 is pressed (step S215) and the control unit 58 performs additional control operations (step S216).

Since, in the third embodiment, the stomach can be expanded by drinking a fluid, like the first embodiment, sufficient space inside the organ necessary for observation by the capsule endoscope 3 can be secured, more detailed images of the inner wall of the organ can be captured, and the stomach can be observed without missing any part inside the stomach. The capsule endoscope 3 can be rocked randomly to drift at the boundary surfaces (a fluid-fluid surface between the first and second fluid and an air-fluid surface between the first fluid and air space) only by changing the position of the boundary surfaces inside the stomach in combination with postural change of the patient himself (herself). Random rocking of the capsule endoscope 3 can cause changes of imaging regions inside the stomach to be captured by the capsule endoscope 3 and further allow for observation without missing any part inside the stomach. This makes it possible, after examination, to combine a plurality of images captured randomly based on correlations of each image to create an overall image inside the stomach for presentation to a physician, leading to efficient diagnosis.

Since ingesta materials are supplied mechanically and actively based on a specific order in the third embodiment, as described above, it is for the patient to accurately and easily take in a plurality of ingesta materials required for examination in the specific order. Since the order of intake is made accurate, anyone can handle the capsule housing apparatus easily and the examination is simplified. Thus, probabilities of a patient taking in ingesta materials in incorrect order are thereby reduced and examination will be carried out more smoothly, leading to prevention of incorrect examination and efficient examination time. Also, since the order of supplying ingesta materials and amounts of intakes can be controlled arbitrarily in accordance with the physical constitution of the patient or examination purposes in the third embodiment, flexibility in supplying ingesta materials is improved, allowing examination of higher precision. Moreover, one capsule housing apparatus can handle examination of a plurality of patients to make examination more efficient.

In the capsule housing apparatus 50 according to the third embodiment, for example, a function to supply a plurality of fluids or a fluid and a solid such as powder by mixing them inside the capsule housing apparatus 50 may be provided. By providing such a mixing function, concentration of fluids inside the apparatus can be controlled to supply optimum fluids suited to individual patients and thus examination of higher precision can be carried out. Further, temperature of the fluids to be supplied can be controlled. The specific gravity of the fluid can thereby be controlled by concentration or temperature to control drifting conditions of the capsule endoscope in the fluid. If, for example, the fluid is water, the specific gravity increases as the temperature drops so that it becomes easier for the capsule endoscope to drift. The specific gravity of an intake fluid may also be changed by first drinking cold water (for example, at temperature of about 10°C) and then raising the temperature by body temperature.

In order to be able to replenish ingesta materials required to be taken by patients together when refilling capsule housing apparatus 50 with fluids, the capsule housing apparatus 50 may also be configured to be refilled by setting a package like in the first embodiment or the second embodiment in which ingesta materials are housed.

### Modification of the third embodiment

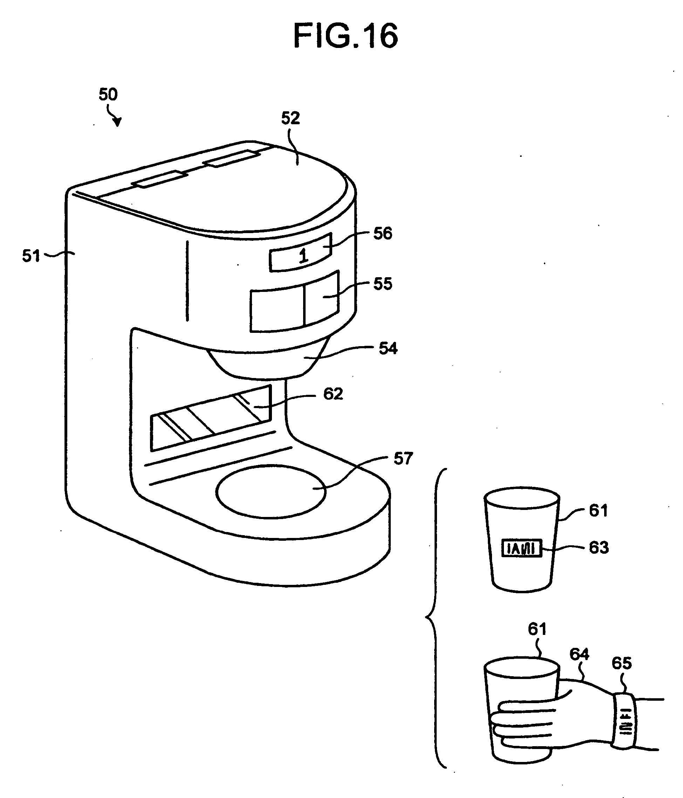

FIG. 16 is a perspective view showing the configuration of a first modification of the capsule housing apparatus 50 according to the third embodiment shown in FIG. 13. In a first modification of the third embodiment, an ID reader 62 that can optically read identification information is provided on the side of the outer frame body 51 and, for example, by reading ID information (for example, black and white pattern information and character information) 63 and 65 stuck to a wrist band attached to the cup 61 or an arm 64 of the patient 1 using the ID reader 62, the capsule housing apparatus 50 (control unit 58) determines which examination to carry out for the patient 1 and in which order to supply fluids, the capsule endoscope 3, and a blowing agent to the patient 1. That is, the control unit 58 is caused to store identification information to identify each patient and examination as ID information and also the orders of examinations based on the ID information, and when the corresponding ID information is read by the ID reader 62, the control unit 58 controls the fluid discharger 54, solid material output port 55, and display unit 56 based on storage content to supply a plurality of ingesta materials in a specific order. Amounts of ingesta materials can also be controlled in accordance with each patient and examination and then supplied. Other components of the first modification of the third embodiment are the same as those of the third embodiment and, for example, the supply button 53 may not be used or may be used in combination with ID reading.

Since, in the first modification of the third embodiment, ID information of a patient is detected using an ID reader and ingesta materials can be made suppliable in a specific order in accordance with the ID information, like the third embodiment, it is for the patient to accurately and easily take in a plurality of ingesta materials required for examination in the specific order. Also, since the order of supplying ingesta materials can be set arbitrarily, the order of supplying fluids and the capsule endoscope 3 can be changed in accordance with examination content, improving flexibility in supplying ingesta materials.

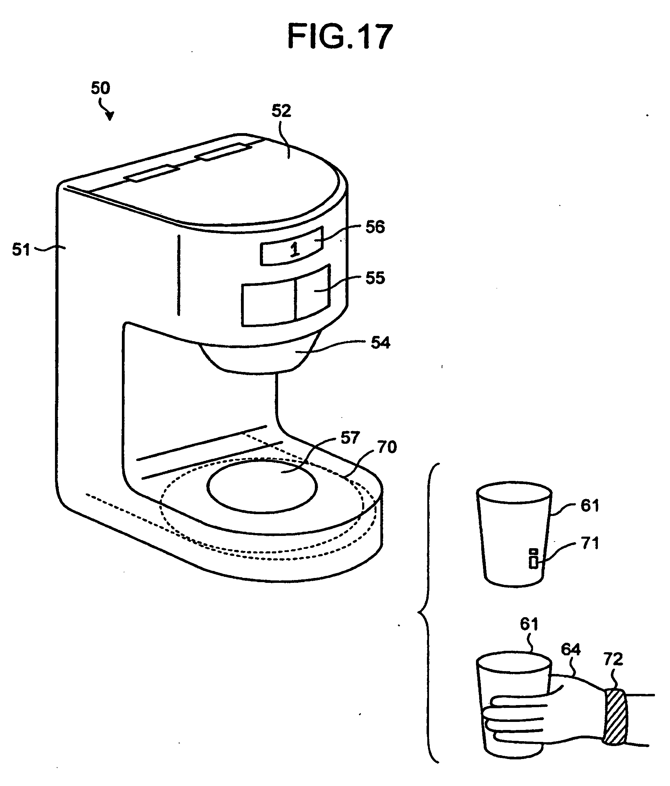

FIG. 17 is a perspective view showing the configuration of a second modification of the capsule housing apparatus according to the third embodiment shown in FIG. 13. In the second modification of the third embodiment, instead of an optical ID reader, a magnetic ID reader 70 that reads ID information from wireless ID tags via radio waves or electromagnetic waves is provided in the capsule housing apparatus 50. Also in this case, by reading ID information from wireless ID tags (RF-ID tags) 71 and 72 stuck to a wrist band attached to the cup 61 or the arm 64 of the patient 1 using the ID reader 70, the capsule housing apparatus 50 (control unit 58) determines which examination to carry out for the patient 1 and in which order to supply fluids, the capsule endoscope 3, and a blowing agent to the patient 1.

Since, in the second modification of the third embodiment, like the first modification of the third embodiment, ID information of a patient is also detected using an ID reader and ingesta materials can be made suppliable in a specific order in accordance with the ID information, like the third embodiment, it is for the patient to accurately and easily take in a plurality of ingesta materials required for examination in the specific order. Also, since the order of supplying ingesta materials amounts of ingesta materials can be set arbitrarily, the order of supplying fluids and the capsule endoscope can be changed in accordance with examination content, improving flexibility in supplying ingesta materials.

Further, in the capsule housing apparatus 50 according to the third embodiment, ingesta materials necessary in accordance with a transmit time of the capsule endoscope 3 (an example of the capsule medical apparatus) taken in by the subject 1 in a living body or other states (the position, posture, or rate of travel of the capsule endoscope 3, or the state of the observation screen or illumination radiation) of the capsule endoscope 3 may be supplied. In this case, the capsule housing apparatus 50 is equipped with a state detector for detecting the transmit time of such a capsule endoscope in a living body or other states of the capsule endoscope, and states (such as the transit time and position in a living body described above) of the capsule endoscope are determined by the state detector.

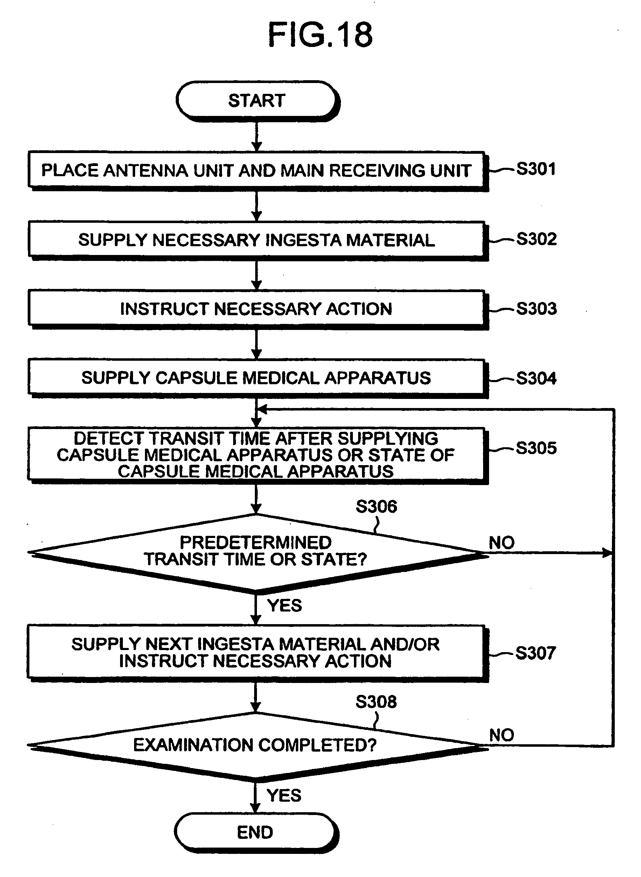

FIG. 18 is an outline flow chart showing another procedure for the method of supplying ingesta materials using the capsule housing apparatus.

As shown in FIG. 18, before supplying ingesta materials to be taken by the subject 1, first the antenna unit 2a having the receiving antennas A1 to An for receiving radio signals from the capsule endoscope 3 is placed at a predetermined position of the subject 1 and the main receiving unit 2b is placed near the subject 1 (step S301).

Next, the supply button 53 of the capsule housing apparatus 50 is operated to supply ingesta materials necessary for the subject 1 at the current moment (step S302). In this case, in response to the operation of the supply button 53, the capsule housing apparatus 50 supplies an ingesta material such as a cleaning agent (remover) for cleaning a digestive tract of the subject 1 to the subject 1. More specifically, such an ingesta material is discharged into the cup 61 or the like to supply it to the subject 1.

Subsequently, after supplying such an ingesta material, the capsule housing apparatus 50 instructs actions necessary for the subject 1 (step S303). In step S303, the capsule housing apparatus 50, for example, instructs the subject 1 to take in the ingesta material such as a remover supplied in step S302 before a capsule medical apparatus such as the capsule endoscope 3 by displaying an instruction in the display unit 56. In this case, the capsule housing apparatus 50 may also instruct the subject 1 who takes in such an ingesta material to change the posture to another posture suitable for taking in the ingesta material when necessary.

Then, the supply button 53 of the capsule housing apparatus 50 is operated again to supply the capsule medical apparatus to be taken by the subject 1 (step S304). In this case, in response to the operation of the supply button 53, the capsule housing apparatus 50 supplies the capsule medical apparatus (an example of the ingesta material) such as the capsule endoscope 3 to be taken by the subject 1 from the solid material output port 55 to the subject 1. The capsule medical apparatus supplied by the capsule housing apparatus 50 will be taken in by the subject 1.

Next, the capsule housing apparatus 50 uses the state detector (not shown) or the like to detect the transit time after supplying the capsule medical apparatus (or after the subject 1 takes in the capsule medical apparatus) or the state of the capsule medical apparatus inside the subject 1 (step S305). In this case, the capsule housing apparatus 50 (more specifically, the state detector) detects such a transit time or a state (for example, the position, posture, or rate of travel of the capsule medical apparatus in the living body, or the state of the observation screen or illumination radiation) of the capsule medical apparatus taken in by the subject 1.

The transit time or the state of the capsule medical apparatus detected by the capsule housing apparatus 50 is a predetermined transit time or state (step S306, Yes), the capsule housing apparatus 50 supplies the next ingesta material necessary next for the subject 1 and/or instructs actions necessary for the subject 1 (step S307). In this case, the capsule housing apparatus 50 supplies the next ingesta material (for example, a fluid such as water) to be taken next by the subject 1 in accordance with a detection result of the transit time or the state of the capsule medical apparatus in step S305. The capsule housing apparatus 50 also displays instruction information in the display unit 56 when necessary to instruct actions (such as intake of the supplied ingesta material or postural change suitable for intake of the ingesta material) necessary for the subject 1.

If the transit time or the state of the capsule medical apparatus detected by the capsule housing apparatus 50 is not the predetermined transit time or state (step S306, No), the capsule housing apparatus 50 returns to step S305 described above and repeats steps from step S305.

Then, if the examination (that is, image capturing or observation of regions to be examined inside the subject 1) for the subject 1 is not completed (step S308, No), the capsule housing apparatus 50 returns to step S305 described above and repeats steps from step S305. If, on the other hand, the examination for the subject 1 is completed (step S308, Yes), the capsule housing apparatus 50 completes the present processing for supplying the ingesta materials to the subject 1.

A concrete example of supplying ingesta materials in accordance with the transit time of the capsule endoscope 3 in a living body will be described below. The capsule housing apparatus 50 supplies a cleaning agent (remover) for cleaning a digestive tract of the subject 1 prior to the capsule endoscope 3 to have the supplied cleaning agent taken in by the patient. To make cleaning of the digestive tract more secure, the capsule housing apparatus 50 may instruct the patient to change the posture by displaying an instruction in the display unit 56 when necessary.

The capsule housing apparatus 50 supplies the capsule endoscope 3 when a predetermined time passes after supplying the cleaning agent or instructing the posture change. The supplied capsule endoscope 3 is taken in by the patient. The capsule housing apparatus 50 stores the time of intake of the capsule endoscope 3 (or the time supplying the capsule endoscope 3). The capsule housing apparatus 50 supplies a fluid, for example, five min. after the time (the intake time or supply time) and prompts the patient to take in the supplied fluid by displaying an instruction in the display unit 56.

Further, the capsule housing apparatus 50 supplies ingesta materials such as other fluids, a blowing agent, a remover, and an antispasmodic each time a predetermined time passes when necessary and instructs intake of the supplied ingesta materials by displaying instructions in the display unit 56. The capsule housing apparatus 50 may also provide instructions such as changing the posture after taking in (after supplying) each ingesta material.

Next, a concrete example of supplying ingesta materials depending on arrival regions (position of the capsule endoscope 3) of the capsule endoscope 3 in a living body and the state of the capsule endoscope 3 will be described. First, the capsule housing apparatus 50 supplies the capsule endoscope 3 with a specific gravity that causes the capsule endoscope 3 to float in a fluid. The supplied capsule endoscope 3 is taken by a patient and then moves in the digestive tract of the patient. The capsule endoscope 3 can make upward (or two directions of upward and downward) observations while drifting in the fluid.

After taking in the capsule endoscope 3, the capsule housing apparatus 50 uses a position detector (an example of the above-described state detector) to check that the capsule endoscope 3 has reached the stomach of the patient, and then supplies a fluid (for example, 200 ml of water), which is the next ingesta material to be taken by the patient. Based on images captured by the capsule endoscope 3, the position detector of the capsule housing apparatus 50 determines the distance between the capsule endoscope 3 and the wall of stomach based on, for example, brightness of such images. If, as a result of state determination processing by the position detector, the distance turns out to be insufficient (distant), since suitable observations cannot be made inside the stomach, the capsule housing apparatus 50 further supplies 100 ml water based on the result of the state determination processing. Additional supply processing of water by the capsule housing apparatus 50 continues until the position detector determines that the distance between the capsule endoscope 3 and the wall of stomach has become appropriate. As a result, the capsule housing apparatus 50 can supply an amount of water appropriate for the patient.

The capsule housing apparatus 50 may supply a blowing agent to facilitate extension of the stomach when necessary. Further, the capsule housing apparatus 50 may provide instructions such as changing the posture necessary for observation of the stomach by displaying such instructions in the display unit 56.

To make minute observations of the patient, the capsule housing apparatus 50 supplies a magnet (a permanent magnet or an electromagnet) for guiding the capsule endoscope 3 introduced into the body of the patient from outside the body. The capsule endoscope 3 inside the body is guided by the supplied magnet and, at the same time, captures images inside the organ successively and, as a result, minute observations inside the body of the patient can be made.

If, after the stomach has been observed, the position detector confirms that the capsule endoscope 3 has reached the duodenum, the capsule housing apparatus 50 supplies 1000 ml of a fluid (such as unabsorbent irrigation water for intestinal lavage) to promote forward movement of the capsule endoscope 3. In this case, the capsule housing apparatus 50 may also supply drugs such as a purgative. Then, if it is confirmed that the capsule endoscope 3 in the body is positioned between jejunum and ileum after taking in such a fluid or a purgative, the capsule housing apparatus 50 further supplies a fluid or a drug for promoting forward movement. The capsule housing apparatus 50 may be configured to have a speed detector (an example of the state detector) for detecting the rate of travel of the capsule endoscope 3 in the body and, in accordance with the rate of travel of the capsule endoscope 3 detected by the speed detector, that is, if the rate of travel falls, further to supply a fluid or a drug for promoting forward movement.

Then, if the position detector detects that the capsule endoscope 3 in the body has reached the large intestine, the capsule housing apparatus 50 supplies an ingesta material (such as a fluid or a purgative) for promoting forward movement and, at the same time, instructs the patient to assume a lie position by displaying such an instruction in the display unit 56. In this case, the capsule housing apparatus 50 may instruct to change the posture of the patient in accordance with the region (such as ascending colon, transverse colon, descending colon, and sigmoid colon) in the large intestine where the capsule endoscope 3 is positioned. Lastly, the capsule housing apparatus 50 supplies a purgative (suppository) for promoting elimination of the capsule endoscope 3 inside the large intestine.

The position detector for detecting the position of the capsule endoscope 3 may determine the position of the capsule endoscope 3 based on features or variations of images captured by the capsule endoscope 3, the position or posture of the capsule endoscope 3 by detecting a magnetic field generated by a magnetic field generator (such as a permanent magnet, coil, and electromagnet) mounted in the capsule endoscope 3 from outside the body, or the position of the capsule endoscope 3 based on received electric-field strength when receiving radio waves transmitted by the capsule endoscope 3 outside the body. The position detector may also be placed outside the body of the patient instead of incorporating into the capsule housing apparatus, and information communication between the position detector and capsule housing apparatus may be performed using wire communication or radio communication. Thus, necessary ingesta materials are supplied in necessary timing or actions such as instructions on postural change are performed and, as a result, examinations inside the body of the patient will be performed more reliably. The state detector is provided in the capsule housing apparatus in the description above, but is not limited to the capsule housing apparatus and may be provided in the receiving device or display unit, or as a single device.

### INDUSTRIAL APPLICABILITY

A capsule housing apparatus according to the present invention and a method of supplying a capsule using the capsule housing apparatus described above are useful in supplying ingesta materials such as a capsule medical apparatus and fluids necessary for examination of a subject, and particularly suitable for a capsule housing apparatus that is made capable of making a subject to take such ingesta materials accurately and easily in a specific order and a method of supplying a capsule using such a capsule housing apparatus.

## Claims

1. A capsule housing apparatus (6; 30; 40; 50), comprising:
a capsule medical apparatus (3) to be introduced into a subject (1);
ingesta materials to be taken by the subject (1), the ingesta materials including at least one of drinkable fluid (7, 8) and a blowing agent for expanding a luminal organ of the subject (1); and
a housing unit (10; 20; 31; 40; 51) having a plurality of housing areas (11, 12; 21, 22; 21a to 21c, 22a, 22b; 31a to 31c; 41, 42; 54, 55), wherein the capsule medical apparatus (3) and at least one of the ingesta materials are each housed in one of the housing areas (11,12; 21, 22; 21a to 21c, 22a, 22b; 31a to 31c; 41, 42; 54, 55) to make the capsule medical apparatus (3) and the ingesta materials suppliable to the subject (1) in a specific order, wherein
the housing unit (31) further comprises a housing area (31a to 31c), which is adapted to house a remover for removing mucus and foams inside the luminal organ, and
the housing unit (31) is adapted to house both the fluid (7, 8) and the remover in the same housing area.

2. The capsule housing apparatus (6; 30; 40; 50) according to claim 1, wherein the fluid (7, 8) is a cleaning fluid or water.

3. The capsule housing apparatus (6; 30; 40; 50) according to claim 1, wherein the ingesta materials contain at least two fluids (7, 8) having at least two different temperatures, and
the at least two fluids (7, 8) are separately suppliable to the subject (1) in the specific order.

4. The capsule housing apparatus (6; 30; 40; 50) according to claim 1, wherein the ingesta materials contain at least two fluids (7, 8) having at least two different concentrations, and
the at least two fluids (7, 8) are separately suppliable to the subject (1) in the specific order.

5. The capsule housing apparatus (6; 30; 40; 50) according to claim 1, wherein the ingesta materials contain at least two fluids (7, 8) having at least two different specific gravities, and
the at least two fluids (7, 8) are separately suppliable to the subject (1) in the specific order.

6. The capsule housing apparatus (6) according to claim 1, wherein the housing unit (10; 20) houses the capsule medical apparatus (3) and the fluid (7, 8) in the identical housing area.

7. The capsule housing apparatus (6) according to claim 1, wherein the housing unit (10; 20) divides the at least one fluid (7, 8) and houses the fluid (7, 8) in a plurality of housing areas (11, 12; 21, 22; 21a to 21c, 22a, 22b) for each of the fluids (7, 8) to make each fluid (7, 8) housed by dividing among the plurality of housing areas (11, 12; 21, 22; 21a to 21c, 22a, 22b) suppliable to the subject (1) in the specific order in accordance with an intake amount necessary for the subject (1).

8. The capsule housing apparatus (30) according to claim 1, wherein the capsule medical apparatus (3) comprises a first magnetic material guidable from outside the subject (1), and
the housing unit (31) further comprises a housing area for housing a second magnetic material for guiding the capsule medical apparatus (3) by a magnetic interaction with the first magnetic material.

9. The capsule housing apparatus (30) according to claim 8, wherein the housing unit (31) further comprises a housing area (31a to 31c) for housing a template for instructing a position of the second magnetic material outside the subject (1).

10. The capsule housing apparatus (6; 40) according to claim 1, further comprising:
a partition wall (13; 25a, 25b; 26; 43) that separates each of the housing areas (11, 12; 21, 22; 21a to 21c, 22a, 22b;41, 42) and allows penetration between the housing areas (11, 12; 21, 22; 21a to 21c, 22a, 22b; 41, 42) in the specific order under a predetermined pressure.

11. The capsule housing apparatus (6; 30) according to claim 1, wherein a marking denoting the specific order is marked to each of the housing areas (11, 12; 21, 22; 21a to 21c, 22a, 22b; 31a to 31c).

12. The capsule housing apparatus (50) according to claim 1, further comprising a state detector that detects a state of the capsule medical apparatus (3), wherein
timing for supplying the capsule medical apparatus (3) and the ingesta materials to the subject (1) in the specific order is determined in accordance with a detection result by the state detector.

## Patentansprüche

1. Kapselaufnahmevorrichtung (6; 30; 40; 50) aufweisend:
eine kapselförmige medizinische Vorrichtung (3) zum Einführen in ein Objekt (1);
Ingestamaterialien zur Einnahme durch das Objekt (1), wobei die Ingestamaterialien wenigstens eine trinkbare Flüssigkeit (7, 8) und einen Dehnstoff zum Ausdehnen eines luminalen Organs des Objekts (1) umfassen; und
eine Aufnahmeeinheit (10; 20; 31; 40; 51) mit einer Vielzahl von Aufnahmebereichen (11, 12; 21, 22; 21a bis 21c, 22a, 22b; 31a bis 31c; 41, 42; 54, 55), wobei die kapselförmige medizinische Vorrichtung (3) und wenigstens eines der Ingestamaterialien jeweils in einem der Aufnahmebereiche (11, 12; 21, 22; 21a bis 21c, 22a, 22b; 31a bis 31c; 41, 42; 54, 55) aufgenommen sind, um die kapselförmige medizinische Vorrichtung (3) und die Ingestamaterialien dem Objekt in einer vorgegebenen Ordnung zuführbar zu machen, wobei
die Aufnahmeeinheit (31) ferner einen Aufnahmebereich (31a bis 31c) aufweist, der zum Aufnehmen eines Entferners zum Entfernen von Schleim und Schäumen innerhalb des luminalen Organs ausgebildet ist, und
die Gehäuseeinheit (31) zum Aufnehmen des Fluids (7, 8) und des Entferners in dem gleichen Gehäusebereich ausgebildet ist.

2. Kapselaufnahmevorrichtung (6; 30; 40; 50) nach Anspruch 1, wobei die Flüssigkeit (7, 8) eine Reinigungsflüssigkeit oder Wasser ist.

3. Kapselaufnahmevorrichtung (6; 30; 40; 50) nach Anspruch 1, wobei die Ingestamaterialien wenigstens zwei Flüssigkeiten (7, 8) enthalten, die wenigstens zwei unterschiedliche Temperaturen aufweisen, und
die wenigstens zwei Flüssigkeiten (7, 8) dem Objekt (1) in einer vorgegebenen Order separat zuführbar sind.

4. Kapselaufnahmevorrichtung (6; 30; 40; 50) nach Anspruch 1, wobei die Ingestamaterialien wenigstens zwei Flüssigkeiten (7, 8) mit wenigstens zwei unterschiedlichen Konzentrationen enthalten, und
die wenigstens zwei Flüssigkeiten (7, 8) dem Objekt (1) in einer vorgegebenen Ordnung separat zuführbar sind.

5. Kapselaufnahmevorrichtung (6; 30; 40; 50) nach Anspruch 1, wobei die Ingestamaterialien wenigstens zwei Flüssigkeiten (7, 8) mit wenigstens zwei unterschiedlichen Schwerkräften aufweisen, und
die wenigstens zwei Flüssigkeiten (7, 8) dem Objekt in einer vorgegebenen Ordnung separat zuführbar sind.

6. Kapselaufnahmevorrichtung (6) nach Anspruch 1, wobei die Aufnahmeeinheit (10; 20) die kapselförmige medizinische Vorrichtung (3) und die Flüssigkeit (7, 8) in dem identischen Aufnahmebereich aufnimmt.

7. Kapselaufnahmevorrichtung (6) nach Anspruch 1, wobei die Aufnahmeeinheit (10; 20) die wenigstens eine Flüssigkeit (7, 8) unterteilt und das Fluid (7, 8) in einer Vielzahl der Aufnahmebereiche (11, 12; 21, 22; 21a bis 21c, 22a, 22b) für jede Flüssigkeit (7, 8) aufnimmt, um jede Flüssigkeit (7, 8), die unterteilt in der Vielzahl der Aufnahmebereiche (11, 12; 21, 22; 21a bis 21c, 22a, 22b) aufgenommen sind, dem Objekt in einer vorbestimmten Ordnung nach Maßgabe einer für das Objekt notwendigen Einnahmemenge zuführbar sind.

8. Kapselaufnahmevorrichtung (30) nach Anspruch 1, wobei die kapselförmige medizinische Vorrichtung (3) wenigstens ein erstes magnetisches Material aufweist, das von der Außenseite des Objekts (1) führbar ist, und
die Aufnahmeeinheit (31) ferner einen Aufnahmebereich zum Aufnehmen eines zweiten magnetischen Materials zum Führen der kapselförmigen medizinischen Vorrichtung (3) durch eine magnetische Interaktion mit dem ersten magnetischen Material aufweist.

9. Kapselaufnahmevorrichtung (30) nach Anspruch 8, wobei die Aufnahmeeinheit (31) ferner einen Aufnahmebereich (31a bis 31c) zum Aufnehmen einer Vorlage zum Anweisen einer Position des zweiten magnetischen Materials außerhalb des Objekts (1) aufweist.

10. Kapselaufnahmevorrichtung (6; 40) nach Anspruch 1, ferner aufweisend:
eine Trennwand (13; 25a, 25b; 26; 43), die jeden der Aufnahmebereiche (11, 12; 21, 22; 21a bis 21c, 22a, 22b; 41, 42) trennt, und die den Durchgang zwischen den Aufnahmebereichen (11, 12; 21, 22; 21a bis 21c, 22a, 22b; 41, 42) in einer vorgegebenen Ordnung unter einem vorbestimmten Druck zulässt.

11. Kapselaufnahmevorrichtung (6; 30) nach Anspruch 1, wobei eine eine vorgegebene Ordnung anzeigende Markierung an jedem der Aufnahmebereiche (11, 12; 21, 22; 21a bis 21c, 22a, 22b; 31a bis 31c) vorgesehen ist.

12. Kapselaufnahmevorrichtung (50) nach Anspruch 1, ferner aufweisend einen Zustandsdetektor, der einen Zustand der kapselförmigen medizinischen Vorrichtung (3) erfasst, wobei die Zeitgabe zum Zuführen der kapselförmigen medizinischen Vorrichtung (3) und der Ingestamaterialien zu dem Objekt (1) in der vorgegebenen Ordnung nach Maßgabe mit dem Erfassungsergebnis des Zustandserfassers festlegbar ist.

## Revendications

1. Appareil de logement de capsule (6 ; 30 ; 40 ; 50), comprenant :
un appareil médical de type capsule (3) destiné à être introduit dans un sujet (1) ;
des matières à ingérer destinées à être prises par le sujet (1), les matières à ingérer incluant au moins un parmi un fluide buvable (7, 8) et un agent gonflant pour dilater un organe luminal du sujet (1) ; et
une unité de logement (10 ; 20 ; 31 ; 40 ; 51) ayant une pluralité de zones de logement (11, 12 ; 21, 22 ; 21a à 21c ; 22a, 22b ; 31a à 31c ; 41, 42 ; 54, 55), dans lequel l'appareil médical de type capsule (3) et au moins une des matières à ingérer sont chacun logés dans une des zones de logement (11, 12 ; 21, 22 ; 21a à 21c ; 22a, 22b ; 31a à 31c ; 41, 42 ; 54, 55) pour faire en sorte que l'appareil médical de type capsule (3) et les matières à ingérer puissent être administrés au sujet (1) dans un ordre spécifique, dans lequel
l'unité de logement (31) comprend en outre une zone de logement (31a à 31c), qui est adaptée à loger un dissolvant pour éliminer un mucus et des mousses à l'intérieur de l'organe luminal, et
l'unité de logement (31) est adaptée à loger à la fois le fluide (7, 8) et le dissolvant dans la même zone de logement.

2. Appareil de logement de capsule (6 ; 30 ; 40 ; 50) selon la revendication 1, dans lequel le fluide (7, 8) est un fluide de nettoyage ou de l'eau.

3. Appareil de logement de capsule (6 ; 30 ; 40 ; 50) selon la revendication 1, dans lequel les matières à ingérer contiennent au moins deux fluides (7, 8) ayant au moins deux températures différentes, et
les au moins deux fluides (7, 8) peuvent être administrés séparément au sujet (1) dans l'ordre spécifique.

4. Appareil de logement de capsule (6 ; 30 ; 40 ; 50) selon la revendication 1, dans lequel les matières à ingérer contiennent au moins deux fluides (7, 8) ayant au moins deux concentrations différentes, et
les au moins deux fluides (7, 8) peuvent être administrés séparément au sujet (1) dans l'ordre spécifique.

5. Appareil de logement de capsule (6 ; 30 ; 40 ; 50) selon la revendication 1, dans lequel les matières à ingérer contiennent au moins deux fluides (7, 8) ayant au moins deux densités différentes, et
les au moins deux fluides (7, 8) peuvent être administrés séparément au sujet (1) dans l'ordre spécifique.

6. Appareil de logement de capsule (6) selon la revendication 1, dans lequel l'unité de logement (10 ; 20) loge l'appareil médical de type capsule (3) et le fluide (7, 8) dans la même zone de logement.

7. Appareil de logement de capsule (6) selon la revendication 1, dans lequel l'unité de logement (10 ; 20) divise l'au moins un fluide (7, 8) et loge le fluide (7, 8) dans une pluralité de zones de logement (11, 12 ; 21, 22 ; 21a à 21c ; 22a, 22b) pour chacun des fluides (7, 8) pour faire en sorte que chaque fluide (7, 8) logé en divisant parmi la pluralité de zones de logement (11, 12 ; 21, 22 ; 21a à 21c ; 22a, 22b) puisse être administré au sujet (1) dans l'ordre spécifique conformément à une quantité d'ingestion nécessaire pour le sujet (1).

8. Appareil de logement de capsule (30) selon la revendication 1, dans lequel l'appareil médical de type capsule (3) comprend un premier matériau magnétique guidable de l'extérieur du sujet (1), et
l'unité de logement (31) comprend en outre une zone de logement pour loger un deuxième matériau magnétique pour guider l'appareil médical de type capsule (3) par une interaction magnétique avec le premier matériau magnétique.

9. Appareil de logement de capsule (30) selon la revendication 8, dans lequel l'unité de logement (31) comprend en outre une zone de logement (31a à 31c) pour loger un modèle pour indiquer une position du deuxième matériau magnétique à l'extérieur du sujet (1).

10. Appareil de logement de capsule (6 ; 40) selon la revendication 1, comprenant en outre :
une cloison (13 ; 25a, 25b ; 26 ; 43) qui sépare chacune des zones de logement (11, 12 ; 21, 22 ; 21a à 21c ; 22a, 22b ; 41, 42) et permet une pénétration entre les zones de logement (11, 12 ; 21, 22 ; 21a à 21c ; 22a, 22b ; 41, 42) dans l'ordre spécifique sous une pression prédéterminée.

11. Appareil de logement de capsule (6 ; 30) selon la revendication 1, dans lequel une marque dénotant l'ordre spécifique est marquée sur chacune des zones de logement (11, 12 ; 21, 22 ; 21a à 21c ; 22a, 22b ; 31a à 31c).

12. Appareil de logement de capsule (50) selon la revendication 1, comprenant en outre un détecteur d'état qui détecte un état de l'appareil médical de type capsule (3), dans lequel
l'instant d'administration de l'appareil médical de type capsule (3) et des matières à ingérer au sujet (1) dans l'ordre spécifique est déterminé en fonction d'un résultat de détection par le détecteur d'état.
